# FASCICULE DE BREVET EUROPEEN

(11) **EP 0 501 882 B1**
(45) Date de publication et mention de la délivrance du brevet: **12.07.2000**
(21) Numéro de dépôt: 92400496.3
(22) Date de dépôt: 26.02.1992
(51) Int. Cl.: C07K 7/06, A61K 39/00, A61K 39/385, A61K 39/395

(54) **Procédé d'immunoneutralisation anti-LHRM des animaux domestiques mâles non castrés et peptide pour cela**
Verfahren zur Immunoneutralisierung von nichtkastrierten männlichen Haustieren gegen LMRH und Peptide dafür
Method of anti LHRH immunoneutralization of non-castrated male animals and peptide therefore

(30) Priorité: 01.03.1991 FR 9102513; 10.12.1991 FR 9115289
(43) Date de publication de la demande: 02.09.1992
(73) Titulaire: MERIAL, 69002 Lyon (FR)
(72) Inventeur: Dufour, Raymond, F-69006 Lyon (FR); Roulet, Claude, F-69200 Venissieux (FR); Chouvet, Claire, F-69007 Lyon (FR); Bonneau, Michel Bernard, F-35160 Montfort (FR)
(74) Mandataire: Bernasconi, Jean

(56) Documents cités:
- EP-A- 0 181 236
- WO-A-88/00056
- WO-A-88/05308
- WO-A-90/11298
- US-A- 4 556 555
- AMERICAN JOURNAL OF PHYSIOLOGY, vol. 242, 1982, pages 201-205; B.D. SCHANBACHER et al.: "Responses of ram lambs to active immunization against testosterone and luteinizing hormone-releasing hormone"
- IDEM
- JOURNAL OF ANIMAL SCIENCE, vol. 63, 1986, pages 986-994; R.E. FALVO et al.: "Effect of active immunization against LHRH or LH in boars: reproductive consequences and performance traits"
- INRA PROD. ANIM., vol. 1, no. 2, 1988, pages 133-140; M. BONNEAU: "Intérêt et limites de la production de viandes de porc mâle entier"

## Description

La présente invention concerne un procédé de production de viande de porcin, bovin, ovin, présentant des qualités organoleptiques, en particulier l'odeur, la sapidité et la tendreté, améliorées à partir d'animaux domestiques mâles non castrés.

L'invention concerne aussi un ensemble de vaccination y relatif.

Les avantages de l'utilisation du mâle entier sur le mâle castré dans l'engraissement des animaux domestiques destinés à la production de viande ont été soulignés depuis plusieurs décennies par les zootechniciens. Ils concernent un taux de croissance plus élevé, surtout chez les bovins et les ovins, une meilleure utilisation de la ration alimentaire et une carcasse plus maigre mais plus fournie en masse musculaire chez toutes les espèces domestiques (S.C. SEIDEMAN et al. J., of Animal Science, 1982, 55 (4) 826-840 et M. BONNEAU, INRA Prod. Anim., 1988, 1 (2) 133-14O).

Les inconvénients principaux de cette utilisation du mâle entier, rappelés dans les revues citées ci-dessus, concernent l'odeur et la sapidité désagréables chez les porcins et ovins mâles, la moindre tendreté de la viande des bovins et ovins mâles entiers et justifient les pratiques actuelles de la castration chirurgicale.

En effet, si les stéroïdes androgènes dont l'androsténediol, l'androsténedione et la testostérone sont les éléments déterminants des avantages attendus dans toutes les espèces domestiques pour une croissance plus rapide et une meilleure utilisation de la ration alimentaire, ils sont rendus responsables d'une moindre tendreté de la viande des bovins et ovins mâles entiers. Les stéroïdes non androgènes ou les dérivés des 16-androstènes dont la 5 α-androsténone (5α-androstèn-16 one-3), chez le porc mâle, sont responsables en partie de l'odeur et de la sapidité désagréables de la viande d'un certain nombre de porcins mâles entiers dès la puberté, lesquelles déprécient la viande et sont un obstacle à sa commercialisation à l'état frais.

Le scatole, produit dérivé du tryptophane et produit par la flore microbienne intestinale, est un composé responsable en partie de l'odeur et de la sapidité désagréables de la viande du porc mâle entier. Sa production dépend de facteurs de l'environnement, de la nutrition, de la race. Son accumulation dans le tissu adipeux est plus importante chez le verrat et serait liée aux sécrétions des stéroïdes sexuels gonadiques.

A titre expérimental, on a déjà essayé de diminuer ou de supprimer le développement du caractère mâle chez le jeune animal ou la sécrétion d'hormones testiculaires, notamment des stéroïdes testiculaires, par immunoneutralisation active ou passive contre ceux-ci ou contre les hormones intervenant dans leur sécrétion, notamment l'hormone lutéinisante ou LH (Luteinizing Hormone) et l'hormone gonadolibérine (GnRH) encore appelée Luteinizing Hormone Releasing Hormone (LHRH). Des essais ont été conduits sur le porc pour abaisser le taux tissulaire de la 5 α-androsténone, du groupe des 16-androstènes, par l'immunisation active dirigée contre ce composé (E.D. WILLIAMSON et al., Liverstock Production Science, 1985, 12, 251-264) ou par l'immunisation passive contre ce même composé (R. CLAUS, Immunization with Hormones in Reproduction Research, ed. Nieschlag, 1975). La suppression ou la diminution de la sécrétion des stéroïdes testiculaires peut être recherchée par l'immunoneutralisation de l'hormone gonadotrope LH, spécifique de l'espèce considérée (R.E. FALVO et al., J. Anim. Science, 1986, 63, 986-994) ou par l'immunoneutralisation anti-LHRH de la LHRH endogène. Seule l'immunisation active anti-LHRH ,a été préconisée par différents auteurs. Chez le porc, l'abaissement de l'α-androsténone a été obtenu par cette méthode (A. CARATY et M. BONNEAU, C.R. Acad. Sci. Paris 1986, 303, Série III (16) 673-676 ; R.E. FALVO et al., J. Anim. Sci., 1986, 63, 986-994).

Chez le mouton, B.D. SCHANBACHER (Am. J.Physiol., 1982, 242, E201-E205) préconise l'immunisation anti-LHRH pour retarder le développement testiculaire et produire un effet de castration chez les agneaux mâles. Chez les bovins, P.S. ROBERTSON (Vet. Rec., 1979, 105, 516-517) décrit une castration immunologique anti-LHRH.

Les essais d'immunoneutralisation anti-LHRH décrits sur les animaux de laboratoire (ARIMURA et al. Endocrinology, 1973, 93, 1092-1103 ; FRASER H.M. et al., J. Endocr. 1974, 63, 399-406 ; MAKINO T. et al. Contraception, 1973, 8 (2), 133-145 ; CARELLI C. et al., Proc. Natl. Acad. Sci., USA, 1982, 79, 5392-5395) et sur plusieurs espèces domestiques (JEFFCOATE et al., Theriogenology, 1978, 10(4), 323-335 ; ROBERTSON I.S. et al., Veterinary Record.,1979, 105, 556 ; SCHANBACHER B.D. Am. J. Physiol., 1982, 242, E201-E205) ont montré qu'il est possible d'obtenir l'arrêt de la sécrétion de la testostérone, l'involution pondérale des testicules et de ses glandes annexes, l'arrêt de la spermatogénèse et, sur le plan du comportement, la disparition de la libido.

Ces travaux ont conduit à suggérer le recours à une immunoneutralisation, notamment anti-LHRH, précoce pour remplacer la traditionnelle castration chirurgicale à des fins d'élevage.

Dans le brevet US n° 4.556.555, il est ainsi décrit une méthode d'immunisation passive d'animaux avant leur puberté, à l'aide d'un antisérum contenant des anticorps dirigés contre la gonadotropine.

La demande de brevet internationale WO 90/11298 décrit un procédé d'immunisation anti-LHRH à la naissance à l'aide de 2 séquences de LHRH en tandem couplées à une protéine porteuse, pour améliorer la qualité de la viande chez le porc.

La demande de brevet internationale WO 88/00056 décrit une méthode de castration immunologique anti-LHRH destinée à améliorer le comportement social et sexuel des animaux mâles en remplacement de la castration chirurgicale qui affecte le taux de croissance. Les taureaux sont vaccinés à l'âge de 8 à 40 semaines et reçoivent ensuite plusieurs rappels.

Un vaccin anti-LHRH vendu sous la marque VAXSTRATE par la société australienne WEBSTERS est utilisé chez la vache.

R.E. Falvo et al. (J. Anim. Sci. 1986, 63 : 986-994) ont immunisé plusieurs groupes de verrats à l'aide de conjugués LHRH-séroglobuline humaine en adjuvant complet de Freund ou avec le muramylpeptide comme adjuvant. Les auteurs ont observé, après vaccination et plusieurs rappels, des titres élevés d'anticorps anti-LHRH, mais avec la nécessité de pratiquer des rappels répétés pour maintenir le titre élevé en anticorps.

I.S. Robertson décrit une méthode d'immunisation avec LHRH conjuguée à l'anatoxine tétanique ou à la thyroglobuline et suggère que l'approche immunologique autoriserait une castration tardive avec les avantages que l'on peut en attendre sur le plan de la croissance pondérale. Il conclut cependant que des efforts sont encore à faire pour arriver à une méthode de castration utilisable dans la pratique, que ce soit au niveau de la méthode elle-même ou de l'adjuvant, l'adjuvant de Freund étant proscrit en pratique.

Enfin, A. Caraty et M. Bonneau (C.R. Acad. Sc. Paris, t. 303, Série III, n°16, 1986) ont pratiqué une immunisation anti-LHRH chez le porc mâle. Les auteurs suggèrent que le blocage de la production de steroïdes, 2 à 3 semaines avant l'abattage, permettrait d'exploiter les potentialités élevées de ce type d'animal pour la production de viande en évitant les problèmes posés par l'accumulation d'androstérone dans le tissu adipeux. Ils concluent cependant que d'importants progrès restent à accomplir dans les techniques d'immunisation avant qu'il ne soit possible de proposer l'immunisation active anti-LHRH comme technique utilisable en élevage porcin.

Par ailleurs, l'immunoneutralisation tardive pose dans la pratique le problème important de l'innocuité du traitement et notamment des réactions locales engendrées par les vaccins, en particulier les vaccins huileux, avec les risques de rejet ou de déclassement de la viande qui en résultent.

L'amélioration des qualités organoleptiques chez les bovins et les ovins n'a pas été suggérée.

La déposante a justement trouvé un procédé applicable industriellement permettant d'améliorer les propriétés organoleptiques de la viande des animaux.

La présente invention a ainsi pour objet un procédé de production de viande de porcin, bovin, ovin présentant des qualités organoleptiques, en particulier odeur, sapidité et tendreté, améliorées, dans laquelle l'on engraisse des animaux domestiques mâles non castrés, l'on effectue chez les animaux une immunoneutralisation active à l'aide d'un vaccin anti-LHRH, caractérisé en ce que l'on maintient les avantages liés au caractère mâle des animaux pratiquement jusqu'à leur abattage par le fait que, avant ou pendant la période d'engraissement des animaux, on leur administre une fois un vaccin anti-LHRH conçu pour induire une première réponse immunitaire de faible intensité, sans effet notable, ou même mesurable, sur la sécrétion des stéroïdes gonadiques et que, seulement peu avant l'abattage, on leur administre un vaccin anti-LHRH pour induire une immunoneutralisation anti-LHRH produisant la suppression ou l'abaissement significatif de la sécrétion des stéroïdes.

Le vaccin administré en premier lieu et le vaccin administré peu avant l'abattage sont choisis parmi les vaccins en émulsion et les vaccins en adjuvant aqueux.

Selon un premier mode de réalisation préféré de ce procédé, on administre à l'animal un vaccin anti-LHRH, de préférence en émulsion, de préférence pendant ou avant la phase d'engraissement de l'animal, puis, peu avant l'abattage de l'animal, on administre à nouveau un vaccin anti-LHRH. On peut procéder en deux administrations distinctes ou par le biais d'un procédé à libération contrôlée.

Chez le porc, il est particulièrement avantageux d'administrer, avant l'abattage, le vaccin anti-LHRH avec un adjuvant de type aqueux, notamment gel d'hydroxyde d'aluminium et/ou saponine.

Cette administration est effectuée de préférence 15 à 21 jours avant l'abattage.

Au contraire, chez le bovin, et éventuellement chez l'ovin, l'administration précédant l'abattage est faite de préférence avec un adjuvant en émulsion, et de préférence 1 à 2 mois avant l'abattage. Cette administration est effectuée de préférence au moins 4 semaines, et de préférence plusieurs mois, après la première administration.

Dans tous les cas, on préfère, pour le vaccin en émulsion, destiné à la première administration et, chez le bovin, à la deuxième administration, que le vaccin se présente sous forme d'émulsion eau-dans-l'huile. Cependant, d'autres formes d'émulsion sont envisageables.

Ce vaccin, de préférence du type en émulsion, est conçu, selon l'invention, pour l'induction d'une première réponse immunitaire de faible intensité, sans effet notable, ou même mesurable, sur la sécrétion des stéroïdes gonadiques. La formulation en émulsion est préférée, mais les autres formulations sont utilisables dès lors qu'elles produisent ce même effet.

L'administration qui précède l'abattage est faite avec un vaccin formulé pour produire à ce moment la suppression ou l'abaissement significatif de la sécrétion des stéroïdes sans réaction locale ou générale adverse, susceptible de nuire à l'apparence ou à la qualité de la viande.

De façon préférée notamment pour le porc, le conjugué, en solution aqueuse, est mis sous les deux formulations suivantes : la première en émulsion eau-dans-l'huile, stable, faite d'un mélange d'huiles minérales animales ou végétales hautement purifiées et de tensioactifs non ioniques pour l'induction d'une réponse immunitaire de faible intensité, sans effet mesurable sur la sécrétion des stéroïdes,gonadiques, la seconde, non émulsionnée, avec gel d'hydroxyde d'aluminium et saponine, déclenchant une réaction immunitaire rapide et intense se traduisant par la production d'anticorps anti-LHRH neutralisants, suffisante pour entraîner la diminution ou la suppression des stéroïdes gonadiques et la diminution du transport associé de scatole d'origine intestinale.

L'émulsion utilisée est, à la différence de celle qui est obtenue à l'aide de l'adjuvant complet ou incomplet de Freund, une émulsion stable permettant de préparer un vaccin prêt à l'emploi. La réaction inflammatoire cutanée reste très faible et localisée aux points d'administration des deux formulations vaccinales et se traduit sous la forme de papules bien circonscrites à l'examen externe. Son développement interne reste limité au derme superficiel. Elle disparaît sans laisser de granulome apparent au moment de l'abattage des animaux.

Selon un autre mode de réalisation de ce procédé, on administre à l'animal, quelques jours avant l'abattage, notamment 5 à 15 jours avant, du sérum ou du plasma hyperimmun anti-LHRH ou encore des anticorps monoclonaux anti-LHRH.

L'immunisation passive anti-LHRH entrainant la diminution voire la suppression de la sécrétion des stéroïdes androgènes et non androgènes a été obtenue par l'administration intramusculaire de plasma hyperimmun équin. Portée à un niveau suffisant, mesuré par le titre en anticorps LHRH du sérum de l'animal receveur, elle entraîne la diminution de la testostérone plasmatique dès le 3ème jour; maintenue à ce même niveau les 12 jours suivants, elle est suffisante pour entraîner l' abaissement de l'androstérone tissulaire au-dessous de 0,50 microgramme/g, valeur pour laquelle l'odeur désagréable et la sapidité particulière de la viande du porc mâle ne seraient plus perçues par le consommateur. Cette méthode d'immunisation passive a montré que le maintien pendant 12 jours de la diminution significative de la testostérone est suffisant pour abaisser la concentration d'androstérone tissulaire au-dessous du seuil fixé. Cette immunisation passive peut être envisagée par l'emploi d'anticorps monoclonaux anti-LHRH secrétés par les hybridomes ou hétérohybridomes porcins.

Le mode d'administration de ces formulations est de préférence transcutané, notamment à l'aide d'un appareil d'injection sans aiguille, par jet sous pression, notamment selon la demande de brevet FR-A-2.652.257.

Le procédé selon l'invention présente l'avantage important de présenter une parfaite innocuité, notamment de ne pas induire des réactions locales susceptibles d'entraîner le déclassement de la viande.

La réaction inflammatoire cutanée reste localisée aux points d'administration des deux formulations vaccinales et se traduit sous la forme de papules bien circonscrites à l'examen externe. Son développement interne reste limité au derme superficiel. Elle disparaît sans laisser de granulome apparent au moment de l'abattage des animaux. La réaction inflammatoire, limitée dans le temps et aux points d'administration, traduit la tolérance aux deux formulations vaccinales et est obtenue par l'administration transcutanée de celles-ci, effectuée à l'aide d'un injecteur sans aiguille.

L'immunisation anti-LHRH nécessite de conjuguer le peptide LHRH ou un fragment du peptide LHRH, non immunogène dans les conditions économiques de leur emploi, à une protéine immunogène, dite porteuse, par une liaison covalente.

La LHRH ou GnRH, qu'elle soit naturelle ou de synthèse, est composée de 10 acides aminés, numérotés de 1 à 10 en allant de la terminaison amino-terminale à la terminaison carboxy-terminale suivant la formule suivante :

pGlu-His-Trp-Ser-Tyr-Gly-Leu-Arg-Pro-Gly NH2
1 2 3 4 5 6 7 8 9 10

Ces symboles, par convention, représentent : pGlu, acide pyroglutamique ; His ; histidine ; Trp, tryptophane ; Ser, sérine ; Tyr, tyrosine ; Gly, glycine ; Leu, leucine ; Arg, arginine ; Pro, proline.

Les conjugués immunogènes anti-LHRH, décrits par les différents auteurs peuvent être réalisés en ce qui concerne l'haptène avec :
a) la LHRH totale ou modifiée en une ou plusieurs de ses parties pour obtenir la conjugaison amino-terminale, carboxy-terminale ou intermédiaire souhaitée,
b) avec l'un de ses fragments peptidiques composés de 5 à 7 acides aminés modifiés ou non, pour obtenir la conjugaison aminoterminale, carboxyterminale ou intermédiaire souhaitée,
c) avec un agoniste portant un acide aminé substitué, le plus couramment en 6, pour obtenir une conjugaison intermédiaire.

En ce qui concerne la protéine porteuse, la sérumalbumine bovine, la sérumalbumine humaine, la thyroglobuline, l'ovalbumine, les globulines humaine ou équine ont été utilisées.

Ainsi, la demande de brevet européen EP-A-181 236 décrit des conjugués immunogènes comprend un nonapeptide ou un décapeptide incluant une séquence, correspondant aux 8 derniers acides aminés de la molécule LHRH, à laquelle est ajoutée une lysine ou une séquence cystéine-lysine du côté aminoterminal.

D'autre part, la demande de brevet WO 88/05308 divulgue des conjugués constitués à l'aide de fragments de 5, 6 ou 7 acides aminés contigus de la molécule naturelle, dans lesquels chaque fragment inclut l'acide pyroglutamique N-terminal ou le glycinamide carboxyterminal et auxquels un acide aminé ou une séquence d'acides aminés additionnels peuvent être ajoutés à l'extrémité liée à la protéine immunogène.

Les agents de conjugaison utilisés peuvent être classés en trois grandes catégories : les agents d'activation, les agents homobifonctionnels, les agents hétérobifonctionnels. Alors que, pour les agents d'activation, la liaison entre les deux molécules se fait entre deux fonctions déjà présentes, pour les autres, la liaison se fait par l'intermédiaire d'un résidu hydrocarboné appelé ligand.

Parmi les agents d'activation, on peut citer l'acide periodique employé pour oxyder les résidus oligosaccharidiques des glycoprotéines en aldéhydes, sur lesquels réagiront ultérieurement les groupements amine de l'autre molécule entrant dans le conjugué.

Les carbodiimides sont des agents d'activation largement employés pour le couplage d'antigènes sur les protéines et, parmi eux, le plus utilisé est sans doute le chlorhydrate de N-éthyl-N′-(diméthylamino-3 propyl) carbodiimide (EDC) qui permet d'effectuer la réaction en milieu aqueux. Leur action conduit à la formation d'une liaison amide entre un groupement carboxyle d'une protéine, activé de façon intermédiaire sous la forme d'une O-alcoylisourée, et un groupement amine porté par une autre molécule. Leur avantage réside dans leur simplicité d'utilisation.

Les agents homobifonctionnels sont des molécules qui possèdent deux groupements réactifs identiques séparés par une chaîne hydrocarbonée. Parmi eux, citons le glutaraldéhyde, qui réagit sur deux groupements amine primaires, les bisisothiocyanates d'alkyle ou d'aryle, qui réagissent sur les amines primaires et les thiols, la benzidine bisdiazotée, qui copule avec les résidus aromatiques de la tyrosine. Mentionnons pour mémoire les bismaléimides et les bisamidinates. L'inconvénient majeur des agents homobifonctionnels est de mal maîtriser la nature des conjugués formés car ces agents peuvent réagir sur deux molécules de même nature et conduire à la formation d'oligomères ou de polymères.

Pour y remédier, des chimistes ont introduit les agents hétérobifonctionnels, dans lesquels les deux groupements ont des spécificités différentes. Dans le cas général, l'un de ces groupements est un ester du N-hydroxysuccinimide qui, dans des conditions douces, réagit sur les groupements amine libres des protéines pour donner d'une part le N-hydroxysuccinimide et d'autre part la protéine portant pa une liaison covalente amide l'agent de couplage sur lequel se trouve la 2ème fonction. Assez généralement, celle-ci peut réagir sur les thiols apportés par la molécule à coupler, ces thiols étant soit initialement présents dans la molécule sous forme de résidus cystéine (ceux-ci pouvant être constitutifs ou, dans le cas de peptides, introduits intentionnellement lors de la synthèse), soit apportés par des agents tels que l'imino-2 thiolane ou le N-((pyridyl-2)dithio-3 propanoyloxy) succinimide (SPDP), après réduction.

Parmi les possibilités énoncées ci-dessus, on préfère utiliser la LHRH totale. En ce cas, la LHRH naturelle est préférée aux agonistes tels que la (D-Lys⁶)-LHRH par la comparaison de l'activité immunogène des conjugués préparés avec ces deux peptides.

Le carbodiimide est préféré au glutaraldéhyde comme agent de conjugaison de la LHRH de forme naturelle sur l'alpha-globuline.

L'alpha-globuline humaine ou équine, fraction IV-1 ou IV-4, est préférée à la sérumalbumine humaine ou bovine.

De préférence, les vaccins comprennent un même principe actif, comprenant préférentiellement un conjugué alpha-globuline-LHRH ; la LHRH est de préférence sous forme naturelle et l'alpha-globuline d'origine humaine ou équine, notamment fractions IV-1 et/ou IV-4. Le conjugué est de préférence obtenu par addition sur 1 volume de mélange alpha-globuline et LHRH en solution de 2 à 20 mg/ml dans NaCl 0,9 % de 0,5 à 2 volumes de solution de chlorhydrate de N-éthyl-N'-(dimétylamino-3-propyl)carbodiimide (EDC) en solution à 2,5 % dans NaCl 0,9 %. Après agitation, le mélange est laissé une nuit, puis purifié par chromatographie de perméation sur gel.

En ce qui concerne la protéine porteuse, on peut utiliser les sérumalbumines, notamment bovine ou humaine, la thyroglobuline, l'ovalbumine, les globulines humaine ou équine, les anatoxines, notamment l'anatoxine tétanique.

La prédominance de la réponse immunitaire des porcs mâles à la fraction carboxyterminale du peptide LHRH conjugué par le carbodiimide ou de son agoniste [D Lys⁶]-LHRH conjugué par le SPDP sur l'alpha-globuline qui a été observée a conduit à la définition d'un conjugué immunogène anti-LHRH utilisant un peptide avantageux présentant la terminaison carboxy-terminale de la LHRH.

La déposante a trouvé qu'il était très avantageux d'utiliser un conjugué d'un peptide comprenant les 8 derniers acides aminés de la LHRH, soit un décapeptide de formule : 
Trp - Ser - Tyr - Gly - Leu - Arg - Pro - Gly - NH2
3 4 5 6 7 8 9 10
 qui possède une grande activité immunogène sans présenter l'activité hormonale de la LHRH naturelle.

L'invention prévoit donc l'utilisation de conjugués incorporant a peptide (3-10) couplé à une protéine porteuse immunogène parmi celles citées plus haut, l'ovalbumine et l'alpha-globuline équine, notamment fractions IV-1 et/ou IV-4, étant préférées.

Dans l'invention, le carbodiimide est préféré au glutaraldéhyde et aux agents hétérobifonctionnels comme agent de conjugaison du peptide LHRH (3-10) notamment sur l'alpha-globuline équine ou l'ovalbumine.

Dans la préparation préférée de conjugué, la LHRH (3-10) et la protéine porteuse, ovalbumine ou alpha-globuline, sont mises en solution à raison de 2 à 40 mg par ml chacune dans le tampon NaCl 0,1M- acide(N-morpholino)-2 éthane sulfonique 0,1M. Ensuite, 0,5 à 2 volumes de solution de N-éthyl-N'(diméthylamino-3 propyl)carbodiimide à 2,5 % dans le même tampon sont additionnés. Le pH est ajusté par addition de soude 1N. Après agitation, le mélange est laissé une nuit, puis est purifié par chromatographie de perméation sur gel, qui élimine la LHRH (3-10) non couplée, le carbodiimide résiduel et ses produits d'hydrolyse.

L'invention a également pour objet l'immunisation passive anti-LHRH (3-10) conformément au procédé décrit plus haut.

L'invention a encore pour objet un ensemble de vaccination anti-LHRH utilisable pour la production de viande ayant des qualités organoleptiques améliorées à partir de porcins, bovins ou ovins mâles non castrés, cet ensemble de vaccination comprenant :
- un vaccin anti-LHRH conçu pour être administré en premier lieu avant ou pendant la phase d'engraissement des animaux et conçu pour induire une première réponse immunitaire de faible intensité, sans effet notable, ou même mesurable, sur la sécrétion des stéroïdes gonadiques, pour permettre le développement du caractère mâle des animaux, et
- un vaccin anti-LHRH conçu pour être administré après le précédent et seulement peut avant l'abattage, ce vaccin étant conçu pour induire une immunoneutralisation anti-LHRH produisant la suppression ou l'abaissement significatif de la sécrétion des stéroïdes.

Elle a ainsi trait aux ensembles regroupant dans un même emballage un nombre égal de doses de vaccin à administrer avant l'abattage et de vaccin à administrer en première injection. De préférence ces vaccins sont conditionnés sous volume réduit et concentration augmentée pour l'administration par jet transcutané, par exemple selon la demande de brevet français précitée.

L'invention va être maintenant décrite plus en détail à l'aide d'une part d'essais comparant plusieurs produits et procédés de vaccination selon l'invention et d'autre part d'essais ayant montré la prédominance de la réponse immunitaire des porcs mâles à la fraction carboxyterminale du peptide LHRH et de l'essai de vaccination anti-LHRH effectué sur les porcs mâles selon l'invention.

### I - UTILISATION DE LA LHRH TOTALE.

**A.** Plus grande activité immunogène du conjugué maintenant intacte la fraction carboxyterminale la plus étendue du peptide LHRH et choix du conjugué à base de LHRH de formule naturelle de préférence à celui obtenu à l'aide de l'agoniste (D-Lys⁶)-LHRH.

**A1.** - Immunisation anti-LHRH du porc mâle entier et du rat mâle OFA.

La comparaison d'activité de deux vaccins anti-LHRH constitués de conjugués entre la LHRH de forme naturelle (B1 et B2) ou la (D-Lys⁶) -LHRH (A1 et A2) et l'albumine humaine, conjugués obtenus par le carbodiimide en phase aqueuse et le SPDP respectivement, mis dans une émulsion huile-dans-l'eau et administrés par voie intramusculaire chez le porc et par voie sous-cutanée chez le rat, conduit aux conclusions suivantes :
- Activité plus grande du vaccin à base de LHRH de forme naturelle : masse du peptide LHRH conjugué inférieure à celle du peptide (D-Lys⁶)-LHRH conjugué pour un recrutement d'un plus grand nombre d'animaux présentant une réponse immunitaire (tableaux 1 et 3).
- Effet de dose qui se traduit par un recrutement d'un nombre plus élevé d'animaux présentant une réponse immunitaire par un même conjugué (tableau 3).

**A1.1 -** Préparation des conjugués (D-Lys⁶)-LHRH-albumine par le SPDP.

La préparation des conjugués (D-Lys⁶)-LHRH-albumine est réalisée en 3 étapes : préparation de la (N-(pyridyl-2)-dithio-3 propanoyl-D-Lys⁶)-LHRH, préparation de la N-(mercapto-3 propanoyl) albumine, puis couplage.

La (N^{ε} -(pyridyl-2)dithio-3 propanoyl-D-Lys⁶)-LHRH est préparée en faisant réagir un excès de SPDP sur la LHRH, en solution aqueuse (6 moles de SPDP par mole de LHRH), puis, après une nuit à 4°C, en centrifugeant le produit obtenu. Celui-ci est dissous dans l'urée 8M et les groupements (pyridyl-2,)dithio présents sont dosés.

La N-(mercapto-3 propanoyl) albumine est obtenue par action de 0,2 mmole de SPDP sur 1 g d'albumine humaine dissoute dans 100 ml de tampon phosphate 0,1M, puis, après une nuit de contact à 4°C et acidification à pH 6, par réduction par le dithiothréitol. Elle est ensuite purifiée par chromatographie de filtration sur gel. Le dosage des thiols et des protéines fournit le niveau de substitution moyen.

Le couplage est effectué en prenant un groupement (pyridyl-2)dithio pour 1,25 groupement thiol. Le pH est amené à 7-7,5, puis, une heure après, le rendement est déterminé par mesure de la pyridine thione-2 libérée.

Le niveau de substitution moyen s'en déduit. Finalement, le conjugué est purifié par chromatographie et est concentré par ultrafiltration.

**A1.2** - Préparation du conjugué LHRH-albumine par le carbodiimide.

A 300 mg de LHRH et 300 mg d'albumine humaine dissous dans 30 ml de NaCl 0,9 % sont additionnés 1000 mg de chlorhydrate de N-éthyl-N'-(diméthylamine-3 propyl) carbodiimide dissous extemporanément dans 40 ml de NaCl 0,9%. Après agitation, le mélange est laissé une nuit à température ambiante à l'abri de la lumière. Ensuite, il est chromatographié sur un gel de Séphadex G-50 ; les fractions correspondant au conjugué sont recueillies, éventuellement concentrées et congelées.

A partir des fractions contenant la LHRH non couplée est déterminée la quantité de LHRH non couplée et donc le niveau de conjugaison moyen. Celui-ci est reproductible et varie de 8 à 10 mg de LHRH couplée pour 100 mg d'albumine.

A partir des spectres UV du conjugué avant et après chromatographie, sont déduits les rendements de la chromatographie en conjugué et donc la quantité (ou la concentration) de LHRH conjuguée.

**A1.3** - Techniques de dosage

Le titre en anticorps est déterminé selon la technique décrite par JEFFCOATE et al., Acta. Endocr., Copenh., 1974, 75 : 625-635.

La testostérone est dosée directement sur plasma par une technique RIA utilisant le radioligand Testostérone C19-carboxyméthyl éther (¹²⁵I)histamine.

La liaison au peptide marqué est déterminée après marquage à l'iode 125 des différents peptides selon COPPOLAND et al., Endocr., 1979, 104 : 1504-1506 et titrage selon la technique décrite par JEFFCOATE et al., Acta Endocr., Copenh., 1974, 75, 625-635.

**A.1.4** - Illustrations
- Essais sur rats
   . Tableau n° 1 : Réponse anticorps anti-LHRH mesurée par le taux de fixation de LHRH marquée à l'iode¹²⁵
   . Tableau n° 2 : Effet de l'immunisation anti-LHRH sur la concentration de testostérone plasmatique.
   . Posologie
      Vaccins
      - A1: : 50 µg de (D-Lys⁶)-LHRH conjuguée
      - B1: : 12 µg de LHRH conjuguée.
- Essais sur porcs mâles entiers.
   . Tableau n° 3 : Réponse anticorps anti-LHRH mesurée par le taux de fixation de LHRH marquée à l'iode¹²⁵
   . Posologie
      Vaccins
      - A1: : 0,5 mg de (D-Lys⁶)-LHRH conjuguée
      - A2: : 6 mg de (D-Lys⁶-)LHRH conjuguée
      - B1: : 0,15 mg de LHRH conjuguée
      - B2: : 1,20 mg de LHRH conjuguée.

**Tableau 1**

| Réponse anticorps anti-LHRH mesurée par le taux de fixation de LHRH marquée à l'iode¹²⁵ RECHERCHE DES ANTICORPS (% Bo/T) DANS LE SERUM (1/100) CHEZ LE RAT | | | | |
|---|---|---|---|---|
| INJECTIONS | TEMPS (SEM) | GROUPE A1 50µg Dlys6-LHRH/HSA/AE1 | GROUPE B1 12µg LHRH/EDC/HSA/AE1 | TITRE B/T=50% |
| SC | 0 | | | |
| | | - | - | - |
| | | - | - | - |
| | | - | - | - |
| | | - | - | - |
| SC | 4 | 0.0 | 15.4 | <100 |
| | | 7.8 | 7.6 | <100 |
| | | 0.0 | 35.3 | <100 |
| | | 0.0 | 51.1 | 100 |
| | 5 | 4.3 | 91.1 | 1600 |
| | | 14.1 | 85.3 | 980 |
| | | 5.9 | 70.8 | 270 |
| | | 15.8 | 94.9 | 2100 |
| | 6 | 0.0 | 64.3 | 120 |
| | | 11.3 | 67.9 | 220 |
| | | 11.7 | 96.1 | 2400 |
| | | 0.0 | 68.3 | 280 |
| | 7 | 14.5 | 72.5 | 400 |
| | | 0.0 | 92.4 | 2100 |
| | | 0.0 | 64.2 | 200 |
| | | 11.5 | 67.2 | 240 |
| | 8 | 19.6 | 70.4 | 310 |
| | | 0.0 | 93.8 | 3200 |
| | | 9.2 | 68.7 | 2200 |
| | | 0.0 | 38.8 | 310 |

**Tableau 2**

| Effet de l'immunisation anti-LHRH sur la concentration testostérone plasmatique DOSAGE DE LA TESTOSTERONE PLASMATIQUE (NG/ML) CHEZ LE RAT | | | | |
|---|---|---|---|---|
| INJECTIONS | TEMPS (SEM) | TEMOINS | GROUPE A1 150 µg Dlys6-LHRH/PDP/HSA | GROUPE B1 12 µg LHRH/EDC/HSA |
| SC | 0 | 0.40 | - | - |
| | | 0.26 | - | - |
| | | 0.47 | - | - |
| | | 0.00 | - | - |
| SC | 4 | 2.25 | 4.16 | 2.51 |
| | | 1.05 | 5.83 | 2.38 |
| | | 2.34 | 4.43 | 3.63 |
| | | - | 4.17 | 0.58 |
| | 5 | 3.80 | 2.99 | 0.00 |
| | | 1.76 | 2.33 | 0.00 |
| | | 5.05 | 2.85 | 0.00 |
| | | 6.67 | 1.54 | 0.00 |
| | 6 | 2.01 | 3.26 | 0.00 |
| | | 4.47 | 0.09 | 0.00 |
| | | 4.69 | 1.10 | 0.00 |
| | | 1.96 | - | 0.00 |
| | 7 | 2.28 | 1.32 | 0.00 |
| | | 1.92 | 0.89 | 0.00 |
| | | 1.89 | 1.59 | 0.00 |
| | | 1.65 | 2.17 | 0.00 |
| | 8 | 0.97 | 1.75 | 0.00 |
| | | 1.91 | 1.43 | 0.00 |
| | | 2.71 | 1.91 | 0.00 |
| | | 1.22 | 2.33 | 0.00 |

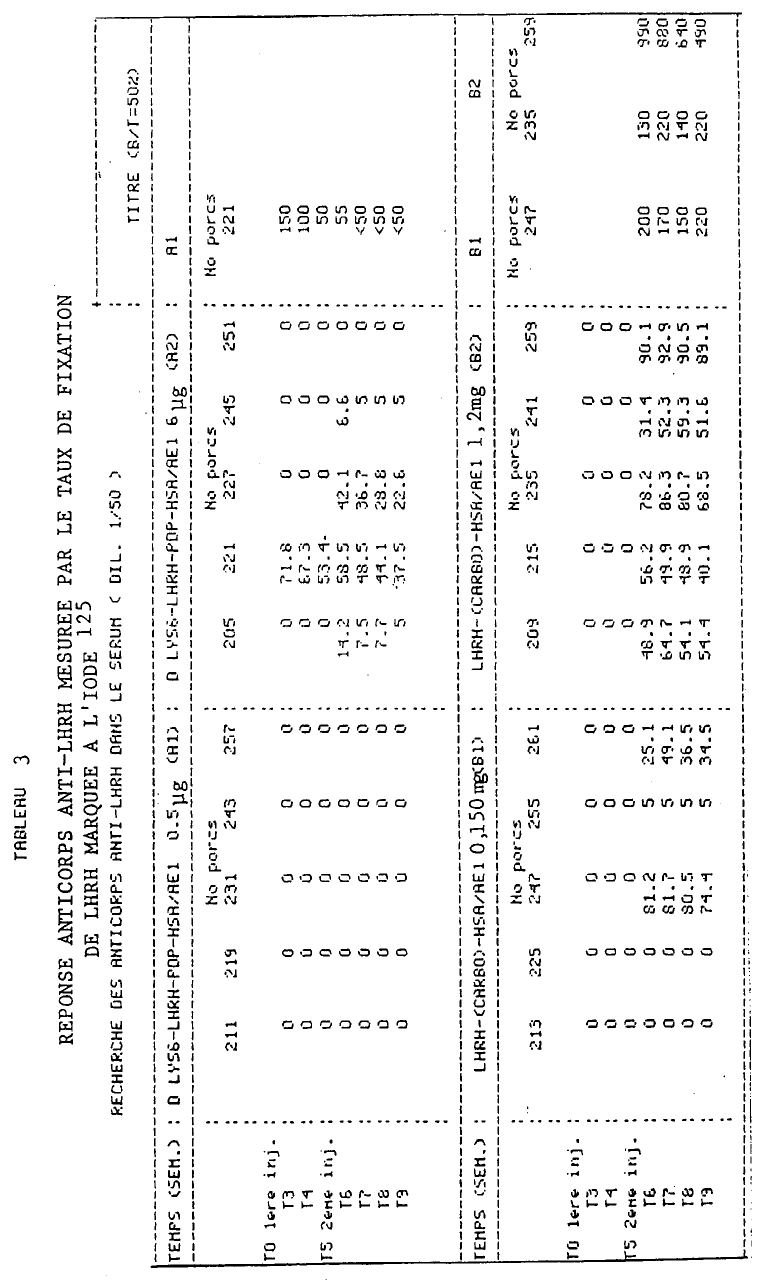

**A2** - Essai comparatif de deux vaccins anti-LHRH composés respectivement d'un conjugué LHRH-α-globuline par le carbodiimide et d'un conjugué (D-Lys⁶)-LHRH-α-globuline par le SPDP mis en émulsion huile-dans-l'eau et administrés par voie intramusculaire (IM) ou transcutanée (ID) chez le porc.

**A2.1** - Préparation de conjugué (D-Lys⁶)-LHRH- α - globuline par le SPDP

La méthode décrite dans l'exemple A1 est employée exactement de la même façon, mais en remplaçant l'albumine à 10 mg/ml par l'α-globuline à 6 mg/ml.

Le rendement global en (D-Lys⁶)-LHRH couplée est de l'ordre de 45 à 50 %.

Par ailleurs il est possible de modifier à volonté le degré de substitution de l'α -globuline, donc le niveau de conjugaison, en jouant sur les concentrations en SPDP et/ou en α -globuline lors de la préparation de la MP- α - globuline.

**A2.2** - Préparation de conjugué LHRH- α -globuline humaine, par le carbodiimide (EDC).

La méthode décrite dans l'exemple A1 est employée exactement de la même façon mais en remplaçant l'albumine humaine par l'α -globuline humaine. Le niveau de conjugaison est de 24 à 28 mg de LHRH fixée pour 100 mg d'α -globuline humaine.

**A2.3** - L'efficacité du vaccin à base du conjugué LHRH- α -globuline par le carbodiimide est supérieure au second. L'efficacité est exprimée par le nombre d'animaux présentant une disparition totale de la testostérone plasmatique (tableau 4).

**Tableau 4**

| | LHRH-α-glob.EDC (1,2 mg) IM + ID | [D-Lys⁶]-LHRH-α-glob.SPDP (6 mg) IM + ID |
|---|---|---|
| Suppression de testostérone | 5/10 | 2/10 |

**A.3** - Prédominance de la réponse immunitaire des porcs mâles à la fraction carboxyterminale du peptide LHRH conjugué par le carbodiimide ou de son agoniste (D-Lys⁶)-LHRH conjugué par le SPDP sur l'α-globuline humaine.

Elle est déterminée par la comparaison des pourcentages de fixation des sérums anti-LHRH et anti-(D-Lys⁶)-LHRH par deux fragments marqués de LHRH, respectivement LHRH (3-10) délété de sa fraction aminoterminale et LHRH (1-10) sous forme d'acide libre et de ce fait délété de la fraction amide de sa fraction carboxyterminale naturelle. Ces deux fractions reconnaissent respectivement plus particulièrement les anticorps dirigés contre les fractions carboxyterminale d'une part, et aminoterminale d'autre part.

La prédominance de la réponse à la fraction carboxyterminale du peptide se traduit par un nombre d'animaux présentant des anticorps ne fixant que le peptide LHRH (3-10) à l'exclusion de la fixation de LHRH acide libre (10/58 pour le sérum anti-LHRH et 3/10 pour le sérum anti-(D-Lys⁶)-LHRH).

Aucun sérum n'a montré de fixation à 100 % de la fraction LHRH acide libre, laquelle traduirait une reconnaissance exclusive de la fraction aminoterminale.

Les réponses mixtes, les plus fréquentes, montrent une meilleure reconnaissance de la fraction aminoterminale par les sérums anti-(D-Lys⁶)-LHRH que celle des sérums anti-LHRH. Chez ces derniers, seuls 3 sérums sur 58 ont une reconnaissance supérieure à 40 % de la fraction aminoterminale, contre 4 sur 10 pour le sérum anti-(D-Lys⁶)-LHRH.

**B** - Plus grande activité immunogène du conjugué LHRH- α -globuline réalisé par le cabodiimide, comparée à celle qui est obtenue par le conjugué préparé avec le glutaraldéhyde.

**B.1** - Préparation du conjugué LHRH- α -globuline par le glutaraldéhyde.

A 10 mg de LHRH et 50 mg d'α -globuline humaine (Serva) dissous dans 5 ml de tampon phosphate 0,1M pH 7,5 sont ajoutés, goutte à goutte et sur une durée de 30 mn, 2,5 ml de solution de glutaraldéhyde à 10 mg/ml, en agitant doucement après chaque addition. Après avoir laissé le mélange 2,5 h à température ambiante, la réaction est arrêtée par addition de 25 mg de bisulfite de sodium dissous dans 0,5 ml d'eau. Le conjugué est dialysé à 4°C contre le tampon NaCl 150mM-phosphate 10mM pH 7,5, puis est concentré par ultrafiltration.

**B.2** - Essai comparatif sur le porc de vaccins anti-LHRH formulés à l'aide de quantités identiques de LHRH conjuguée. L'efficacité est exprimée par le nombre d'animaux présentant une disparition totale de la testostérone plasmatique (tableau 7).

**Tableau 7**

| | LHRH-α-glo. par carbodiimide administration IM ou ID | LHRH-α-glo. par glutaraldéhyde administration IM ou ID |
|---|---|---|
| Suppression de testostérone plasmatique | 5/10 | 0/10 |

**C** - Plus grande activité immunogène du conjugué utilisant l'α-globuline humaine comparée à celle qui est obtenue par le conjugué utilisant de la sérumalbumine humaine.

L'efficacité est exprimée par le nombre d'animaux présentant une disparition totale de la testostérone plasmatique (tableau 8).

**Tableau 8**

| Essais sur porcs - injection intramusculaire | | |
|---|---|---|
| | LHRH-HSA par carbodiimide | LHRH-α-glo. par carbodiimide |
| Suppression de testostérone plasmatique | 0/5 | 3/5 |

**D** - Activité immunogène du conjugué utilisant l'α-globuline équine, fraction IV-1, équivalente à celle qui est obtenue par le conjugué utilisant l'α-globuline humaine.

**D.1** - Préparation de conjugué LHRH-α-globuline équine par le carbodiimide.

La méthode décrite dans l'exemple A1 est employée exactement de la même façon, mais en remplaçant l'albumine humaine par l'α-globuline équine (fraction IV-1).

**D.2** - Administration chez le rat par voie sous-cutanée à 2 reprises à 4 semaines d'intervalle d'un vaccin à la dose de 12 µg de LHRH conjuguée à l'α-globuline humaine ou équine.

**Tableau 9**

| Essais sur rats | | |
|---|---|---|
| | LHRH-α-globuline humaine fraction IV-1 par carbodiimide | LHRH-α-globuline équine fraction IV-1 par carbodiimide |
| Suppression de la testostérone plasmatique | 12/12 | 12/12 |

**E** - Plus grande activité adjuvante de l'émulsion eau-dans-l'huile de l'invention sur d'autres émulsions (tableau 10).

Essais sur le porc en utilisant le même conjugué composé de la LHRH et de l'α-globuline humaine par le carbodiimide et administré à la même dose sous un même volume par la voie transcutanée en 5 points.

Les émulsions examinées sont : une émulsion fluide huile-dans-l'eau (B), l'émulsion de l'invention (formule C du tableau), une émulsion du commerce à diluer avec l'antigène (E), une phase huileuse à émulsionner avec le conjugué (F).

Pour toutes ces formules, la quantité finale d'antigène par dose est la même.

Les émulsions sont réalisées dans les conditions habituelles pour ceux experts en formulation de ce type.

**Tableau 10**

| Emulsions | B | C | E | F |
|---|---|---|---|---|
| Suppression de la testostérone plasmatique | 2/5 | 4/4 | 1/5 | 3/5 |
| Nombre d'animaux présentant une concentration de l'androsténone tissulaire au-dessous de 0,5 µg/g | 2/5 | 4/4 | 3/5 | 3/5 |

**F.** Efficacité de l'immunisation passive anti-LHRH pour l'amélioration des qualités organoleptiques de la viande, mesurée par l'abaissement de l'androsténone tissulaire.

**Tableau 11**

| Teneur en adrosténone du tissu adipeux chez les animaux témoins et chez ceux qui ont été soumis à l'immunoneutralisation anti-LHRH passive par un plasma hyperimmun équin anti-(D-Lys⁶)-LHRH administré sous un volume de 300 ml aux jours 16, 13, 9 et 5 avant abattage. | | |
|---|---|---|
| | Témoins | Traités |
| Nombre d'animaux présentant une concentration d'androsténone intérieure à 0,50 µg/g de tissu adipeux | 2/5 | 5/5 |
| (différence significative au risque α = 0,2) | | |

**G.** - Efficacité et tolérance des formulations renfermant la LHRH conjuguée à l'α-globuline par le carbodiimide en émulsion eau-dans-l'huile (ler vaccin) et en gel d'hydroxyde d'aluminium et saponine (2ème vaccin), administrées à la même dose de LHRH conjuguée, respectivement au début de la mise en engraissement et 18 à 21 jours lavant l'abattage par voie transcutanée à l'aide d'un injecteur sans aiguille dénommé Pigjet.

Deux essais ont été effectués en deux temps, respectivement les groupes 1, 3 et 5 pour le premier et les groupes 2 et 4 pour le second (tableaux 12 et 13).

**G.1** - L'efficacité de l'immunoneutralisation, anti-LHRH est augmentée pour un volume égal de vaccin par la multiplication des points d'administration transcutanée.

**Tableau 12**

| Groupes | 1 | 2 | 3 | 4 | 5 |
|---|---|---|---|---|---|
| 1er vaccin | 1 ml (5 points) | 1 ml (5 points) | 1 ml (5 points) | 0,4 ml (10 points) | 0,4 ml (2 points) |
| 2e vaccin | 1 ml (5 points) | 1 ml (5 points) | 0,4 ml (2 points) | 0,4 ml (10 points) | 0,4 ml (2 points) |
| Suppression ou diminution marquée de la testostérone (nbre d'animaux) | 10/12 | 10/11 | 9/12 | 11/11 | 8/11 |
| Concentration de l'androsténone tissulaire au-dessous de 0,5 µg/g (nbre d'animaux) | 11/12 | ND | 10/23 | ND | ND |
| ND : non déterminé | | | | | |

**G.2** - La tolérance aux vaccins utilisés est jugée par l'évolution de la réaction inflammatoire cutanée, notée de 0 à 4 chez un animal en fonction de l'importance des papules apparaissant après l'administration ; une papule apparaît à chaque point d'administration. La sommation des notes dans chacun des groupes est résumée comme suit : note moyenne à l'issue de la première semaine qui suit l'administration (Ad. 1) et note moyenne au moment de l'abattage pour chacun des vaccins (Ab) (tableau 13). La meilleure tolérance est observée avec l'emploi des vaccins dans le groupe 4.

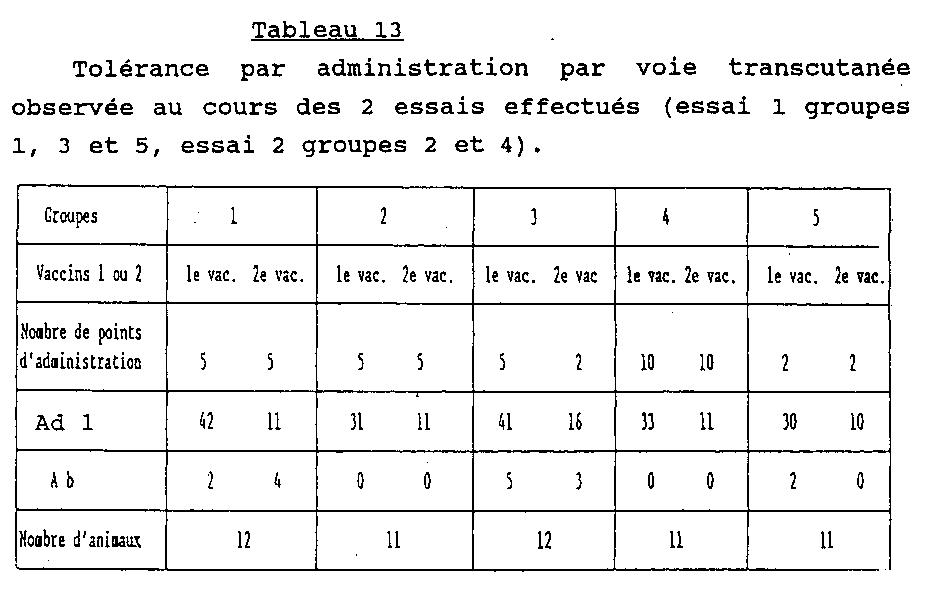

### II - UTILISATION DU PEPTIDE (3-10).

A. Techniques de la mesure de la réponse immunitaire anti-LHRH et de l'efficacité biologique par le dosage de la testostérone plasmatique et de l'androstérone tissulaire.

La réponse immunitaire anti-LHRH est mesurée par le titre en anticorps qui est déterminé selon la technique décrite par JEFFCOATE et al., Acto. Endocr. (Copenh.), 1974, 75, 625-635.

La liaison aux peptides marqués est déterminée après marquage à l'iode 125 des différents peptides selon COPPOLAND et al., Endocrinology., 1979, 104, 1504-1506. Le titrage des sérums vis-à-vis de ces peptides est effectué selon la technique de JEFFCOATE et al. citée ci-dessus.

L'efficacité biologique est mesurée par l'abaissement ou la disparition de la testostèrone plasmatique et de l'androsténone tissulaire. Le dosage de la testostérone plasmatique est effectué directement sur le plasma par une technique RIA utilisant le radioligand testostérone C19-carboxyméthyl éther (¹²⁵I) histamine (FURUYAMA S. et al., Steroids, 1972, 16, 415). Le dosage de l'androsténone tissulaire est effectué sur un échantillon de tissu adipeux par une technique RIA utilisant le radioligand 5α-³H-androsténone, décrite par CLAUS, C.R. Acad. Sci., Paris, 1974, 278, 299-302.

B. Prédominance de la réponse immunitaire des porcs mâles à la fraction carboxyterminale du peptide LHRH conjugué par le carbodiimide ou de son agoniste [D-Lys⁶]-LHRH conjugué par le SPDP sur l'α-globuline humaine.

B1. Préparation du conjugué LHRH-α-globuline humaine par le carbodiimide.

Le conjugué est de préférence obtenu par addition à un volume du mélange α -globuline et LHRH, en solution à 2 à 20 mg/ml dans NaCl 0,9%, de 0,5 à 2 volumes de solution de chlorhydrate de N-éthyl-N′-(diméthylamino-3 propyl)carbodiimide (EDC) en solution à 2,5% dans NaCl 0,9%. Après agitation, le mélange est laissé une nuit, puis purifié par chromatographie de perméation sur gel.

B2. Préparation des conjugués ([D.Lys⁶]-LHRH)-α-globuline humaine par le SPDP.

La préparation des conjugués ([D.Lys⁶]-LHRH)-α-globuline humaine est réalisée en 3 étapes : préparation de la [N-(pyridyl-2)-dithio-3 propanoyl-D-Lys⁶]-LHRH, préparation de la N-(mercapto-3 propanoyl) α-globuline humaine, puis couplage.

La [N^{ε}-(pyridyl-2)dithio-3 propanoyl-D-Lys⁶]-LHRH est préparée en faisant réagir un excès de SPDP sur la LHRH, en solution aqueuse (6 moles de SPDP par mole de [D-Lys⁶]-LHRH, puis, après une nuit à 4°C, en centrifugeant le produit obtenu. Celui-ci est dissous dans l'urée 8M et les groupements (pyridyl-2)dithio présents sont dosés.

La N-(mercapto-3 propanoyl) α-globuline humaine est obtenue par action de 0,2 mmole de SPDP sur 0,6 g d'α-globuline humaine dissoute dans 100 ml de tampon phosphate 0,1M, puis, après une nuit de contact à 4°C et acidification à pH 6, par réduction par le dithiothréitol. Elle est ensuite purifiée par chromatographie de filtration sur gel. Le dosage des thiols et des protéines fournit le niveau de substitution moyen.

Le couplage est effectué en prenant un groupement (pyridyl-2)dithio pour 1,25 groupement thiol. Le pH est amené à 7-7,5, puis, une heure après, le rendement est déterminé par mesure de la pyridine thione-2 libérée.

Le niveau de substitution moyen s'en déduit. Finalement, le conjugué est purifié par chromatographie et est concentré par ultrafiltration. Le rendement global en [D-Lys⁶]-LHRH couplée est de l'ordre de 45 à 50%.

B3. La prédominance de la réponse immunitaire des porcs mâles à la fraction carboxyterminale du peptide LHRH conjugué dans les conditions décrites en A1 et A2 est déterminée par la comparaison de la fixation par les sérums anti-LHRH et anti-[D-Lys⁶]-LHRH, de deux fragments marqués de LHRH, respectivement LHRH (3-10), (LHRH délétée de sa fraction aminoterminale) et LHRH (1-10) sous forme d'acide libre, (LHRH délétée de la fraction amide de sa fraction carboxyterminale). Ces deux fragments reconnaissent plus particulièrement les anticorps dirigés respectivement contre les fractions carboxyterminale d'une part, et aminoterminale d'autre part.

La réponse à la fraction carboxyterminale du peptide est générale sur tous les animaux immunisés par l'un ou l'autre des conjugués (68/68). Les sérums de 3 sur 10 des 10 animaux immunisés par la [D-Lys⁶]-LHRH conjuguée et de 10 sur 68 des animaux immunisés par la LHRH conjuguée ont montré exclusivement une fixation de la fraction carboxyterminale. Les autres animaux présentent une réponse mixte dirigée préférentiellement contre la fraction carboxyterminale.

La réponse à la fraction aminoterminale n'est pas générale (55/68). Aucun sérum n'a montré de fixation exclusive à la fraction LHRH acide libre.

C. Essais d'immunoneutralisation active anti-LHRH à l'aide des conjugués LHRH (3-10)-α-globuline équine IV-4 et LHRH (3-10)-ovalbumine réalisés par le carbodiimide.

C1. Préparation du conjugué LHRH (3-10)-α-globuline équine IV-4 par le carbodiimide.

Quatre-vingt-cinq mg de LHRH (3-10) et 170 mg d'α-globuline équine IV-4 sont dissous dans 12,8 ml de tampon NaCl 0,1M - acide (N-morpholino)-2 éthanesulfonique 0,1M. Puis 212 mg de chlorhydrate de N-éthyl-N′-(diméthylamino-3 propyl)carbodiimide dissous dans 17 ml de solution précédente sont ajoutés. Le pH est immédiatement ajusté à 6,0 par addition de 1,3 ml de soude 1N.

Après agitation, le mélange est laissé 16h à température ambiante, puis le conjugué est purifié par chromatographie de perméation sur gel pour séparer le conjugué de la LHRH non conjuguée. La mesure de la quantité de cette dernière permet d'avoir par différence la quantité de LHRH couplée. Il est possible de déterminer le rendement de couplage.

C2. Préparation du conjugué LHRH (3-10)-ovalbumine par le carbodiimide.

Soixante mg de LHRH (3-10) et 120 mg d'ovalbumine sont dissous dans 9 ml de tampon NaCl 0,1M - acide (N-morpholino)-2 éthanesulfonique 0,1M. Puis 150 mg de chlorhydrate du N-éthyl-N′-(diméthylamino-3 propyl)carbodiimide dissous dans 12 ml du même tampon sont ajoutés. Le pH est ajusté à 7,0 par addition de soude 1N (1,9 ml environ). Le mélange est laissé une nuit à température ambiante, il est ensuite clarifié par centrifugation. Le surnageant est chromatographié sur gel de Séphadex pour séparer le conjugué de la LHRH n'ayant pas réagi et des produits provenant du carbodiimide initial. Par mesure de la quantité de LHRH (3-10) non fixée, il est possible de déterminer le rendement de couplage de la LHRH (3-10).

C3. Réponse immunitaire, efficacité biologique et tolérance au vaccin anti-LHRH formulé à partir du conjugué obtenu entre les fragments LHRH (3-10) et l'α-globuline équine IV-4 par le carbodiimide.

Les formulations constituées de la LHRH (3-10) conjuguée mises en émulsion eau-dans-l'huile (1er vaccin) et en gel d'hydroxyde d'aluminium et saponine (2e vaccin) ont été administrées à 6 porcs mâles sous un volume de 0,4 ml par dose, respectivement au début de la mise en engraissement et 17 jours avant l'abattage par voie transcutanée à l'aide d'un injecteur sans aiguille dénommé Pigjet délivrant le volume de la dose en 2 applications de 0,2 ml réparties en 5 points à chacune d'elles.

La réponse immunitaire fut maximale 10 jours après l'administration du 2e vaccin. Les titres individuels en anticorps (inverse de la dilution par laquelle l'iode 125 est fixé à 50%) furent respectivement :

| | | | | | | |
|---|---|---|---|---|---|---|
| Jour 10 | 280 | 660 | 2 700 | 3 200 | 4 600 | 13 000 |
| Jour 16 | 290 | 400 | 2 000 | 2 400 | 3 100 | 8 600 |

L'efficacité biologique de cette réponse immunitaire s'est traduite par la disparition de la testostérone plasmatique dès le 10e jour après l'administration du 2e vaccin chez tous les 6 animaux. La disparition de la testostérone s'accompagne, dans les mêmes conditions, de la disparition de l'androsténone tissulaire.

La tolérance au vaccin est jugée par l'évolution de la réaction inflammatoire cutanée, notée en fonction de l'importance des papules apparaissant à chaque point de délivrance du vaccin après administration, Cette inflammation locale a totalement disparu dès le jour 10 après l'administration du 2e vaccin.

## Revendications (Revendications pour l'(les) Etat(s) contractant(s) suivant(s): AT, BE, CH, LI, DE, DK, FR, GB, IT, LU, NL, PT)

1. Procédé de production de viande de porcin, bovin, ovin présentant des qualités organoleptiques, en particulier odeur, sapidité et tendreté, améliorées, dans laquelle l'on engraisse des animaux domestiques mâles non castrés, l'on effectue chez les animaux une immunoneutralisation active à l'aide d'un vaccin anti-LHRH, caractérisé en ce que l'on maintient les avantages liés au caractère mâle des animaux pratiquement jusqu'à leur abattage par le fait que, avant ou pendant la période d'engraissement des animaux, on leur administre une fois un vaccin anti-LHRH conçu pour induire une première réponse immunitaire de faible intensité, sans effet notable, ou même mesurable, sur la sécrétion des stéroïdes gonadiques et que, seulement peu avant l'abattage, on leur administre un vaccin anti-LHRH pour induire une immunoneutralisation anti-LHRH produisant la suppression ou l'abaissement significatif de la sécrétion des stéroïdes.

2. Procédé selon la revendication 1, caractérisé en ce que le vaccin administré en premier lieu et le vaccin administré peu avant l'abattage sont choisis parmi les vaccins en émulsion et les vaccins en adjuvant aqueux.

3. Procédé selon l'une quelconque des revendications 1 et 2, caractérisé en ce que le vaccin anti-LHRH administré en premier lieu est un vaccin en émulsion.

4. Procédé selon l'une quelconque des revendications 1 à 3, caractérisé en ce que, pour le porc, on administre, peur avant l'abattage, le vaccin anti-LHRH avec un adjuvant de type aqueux.

5. Procédé selon la revendication 4, caractérisé en ce que, comme adjuvant de type aqueux, on utilise du gel d'hydroxyde d'aluminium et/ou de la saponine.

6. Procédé selon la revendication 4 ou 5, caractérisé en ce qu'on administre le vaccin en adjuvant aqueux de 15 à 21 jours avant l'abattage.

7. Procédé selon l'une quelconque des revendications 1 à 3, caractérisé en ce que, pour les bovins et les ovins, on administre, avant l'abattage, un vaccin anti-LHRH avec un adjuvant en émulsion.

8. Procédé selon la revendication 7, caractérisé en ce qu'on administre le vaccin en émulsion de un à deux mois avant l'abattage.

9. Procédé selon la revendication 7 ou 8, caractérisé en ce qu'on administre le vaccin en émulsion de quatre semaines à plusieurs mois après l'administration faite en premier lieu.

10. Procédé selon l'une quelconque des revendications 2, 3 et 7 à 9, caractérisé en ce que le vaccin en émulsion est un vaccin en émulsion eau-dans-l'huile.

11. Procédé selon la revendication 10, caractérisé en ce que l'émulsion eau-dans-l'huile est faite d'un mélange d'huiles minérales hautement purifiées et de tensioactifs non ioniques.

12. Procédé selon l'une quelconque des revendications 1 à 11, caractérisé en ce que l'on administre un conjugué immunogène anti-LHRH comprenant :
- la LHRH totale ou modifiée,
- un fragment peptidique de LHRH modifié ou non, ou
- un agoniste de la LHRH, couplé à une protéine porteuse immunogène choisie parmi :
- sérum albumine bovine ou humaine,
- thyroglobuline,
- ovalbumine,
- les anatoxines, notamment l'anatoxine tétanique,
- globulines humaines ou équines.

13. Procédé selon la revendication 12, caractérisé en ce que le conjugué comprend la LHRH couplée à l'alpha-globuline équine, notamment fractions IV-1 et/ou IV-4.

14. Procédé selon la revendication 12, caractérisé en ce que le conjugué comprend la LHRH (3-10) couplée à l'alpha-globuline équine, notamment les fractions IV-1 et/ou IV-4, ou à l'ovalbumine.

15. Procédé selon la revendication 13 ou 14, caractérisé en ce que la LHRH/LHRH (3-10) et la protéine porteuse immunogène sont couplées par un carbodiimide.

16. Procédé selon la revendication 1, caractérisé en ce que, au lieu de vaccin pour immunoneutralisation active, on administre à l'animal, quelques jours avant l'abattage, du sérum ou du plasma hyperimmun anti-LHRH.

17. Procédé selon la revendication 1, caractérisé en ce que, au lieu de vaccin pour immunoneutralisation active, on administre à l'animal, quelques jours avant l'abattage, des anticorps monoclonaux anti-LHRH.

18. Procédé selon la revendication 16 ou 17, caractérisé en ce que l'administration est faite de cinq à quinze jours avant l'abattage, par voie sous-cutanée ou intramusculaire.

19. Procédé selon l'une quelconque des revendications 1 à 16, caractérisé en ce qu'on administre par voie transcutanée, de préférence en plusieurs points, à l'aide d'un appareil d'injection sans aiguille par jet sous pression.

20. Procédé selon l'une quelconque des revendications 1 à 19, caractérisé en ce que le vaccin anti-LHRH administré en premier lieu l'est avant la phase d'engraissement de l'animal.

21. Ensemble de vaccination anti-LHRH utilisable pour la production de viande ayant des qualités organoleptiques améliorées à partir de porcins, bovins ou ovins mâles non castrés, cet ensemble de vaccination comprenant :
- un vaccin anti-LHRH conçu pour être administré en premier lieu avant ou pendant la phase d'engraissement des animaux et conçu pour induire une première réponse immunitaire de faible intensité, sans effet notable, ou même mesurable, sur la sécrétion des stéroïdes gonadiques, pour permettre le développement du caractère mâle des animaux, et
- un vaccin anti-LHRH conçu pour être administré après le précédent et seulement peut avant l'abattage, ce vaccin étant conçu pour induire une immunoneutralisation anti-LHRH produisant la suppression ou l'abaissement significatif de la sécrétion des stéroïdes.

22. Ensemble de vaccination selon la revendication 21, caractérisé en ce que les vaccins comprennent un principe actif qui est un conjugué entre une protéine porteuse immunogène et un peptide LHRH.

23. Ensemble de vaccination selon la revendication 22, caractérisé en ce que le peptide LHRH est choisi dans le groupe consistant en :
- LHRH totale ou modifiée,
- fragment peptidique de LHRH, modifié ou non,
- agoniste de LHRH.

24. Ensemble de vaccination selon la revendication 22 ou 23, caractérisé en ce que la protéine porteuse immunogène est choisie dans le groupe consistant en :
- sérum albumine,
- thyroglobuline,
- ovalbumine
- anatoxines,
- anatoxine tétanique,
- globulines équines,
- globulines humaines,
- alpha-globulines.

25. Ensemble de vaccination selon l'une des revendications 21 à 24, caractérisé en ce que le vaccin anti-LHRH à administrer en première injection est un vaccin sous forme d'émulsion.

26. Ensemble de vaccination selon l'une des revendications 21 à 25, caractérisé en ce que le vaccin anti-LHRH à administrer peu avant l'abattage est un vaccin sous forme d'émulsion.

27. Ensemble de vaccination selon la revendication 25 ou 26, caractérisé en ce que le vaccin sous forme d'émulsion est un vaccin sous forme d'émulsion eau-dans-l'huile.

28. Ensemble de vaccination selon l'une des revendications 25 à 27, caractérisé en ce que le vaccin sous forme d'émulsion est fait d'un mélange d'huiles minérales et de tensioactifs non ioniques.

29. Ensemble de vaccination selon l'une des revendications 21 à 25, caractérisé en ce que le vaccin anti-LHRH destiné à être administré peu avant l'abattage est un vaccin en adjuvant de type aqueux.

30. Ensemble de vaccination selon la revendication 29, caractérisé en ce que l'adjuvant de type aqueux est choisi dans le groupe consistant en gel d'hydroxyde d'aluminium, saponine et mélange de ceux-ci.

31. Ensemble de vaccination selon l'une des revendications 21 à 25 et 29, 30, destiné aux porcs, caractérisé en ce que le vaccin anti-LHRH à administrer peu avant l'abattage est un vaccin en adjuvant de type aqueux.

32. Ensemble de vaccination selon l'une des revendications 21 à 28, utilisable chez les bovins et les ovins, caractérisé en ce que le vaccin anti-LHRH à administrer peu avant l'abattage est un vaccin sous forme d'émulsion.

33. Ensemble de vaccination selon l'une quelconque des revendications 21 à 32, caractérisé en ce que l'ensemble de vaccination est constitué d'un nombre identique de doses de chacun des vaccins, dans un emballage unique.

34. Procédé selon l'une quelconque des revendications 1 à 11, caractérisé en ce que l'on administre un vaccin comprenant un principe actif immunogène comprenant le peptide LHRH.

35. Ensemble de vaccination selon la revendication 21 ou selon l'une quelconque des revendications 25 à 33 ensemble la revendication 21, caractérisé en ce que les vaccins anti-LHRH comportent un principe actif immunogène comprenant le peptide LHRH.

## Revendications (Revendications pour l'(les) Etat(s) contractant(s) suivant(s): ES, GR)

1. Procédé de production de viande de porcin, bovin, ovin présentant des qualités organoleptiques, en particulier odeur, sapidité et tendreté, améliorées, dans laquelle l'on engraisse des animaux domestiques mâles non castrés, l'on effectue chez les animaux une immunoneutralisation active à l'aide d'un vaccin anti-LHRH, caractérisé en ce que l'on maintient les avantages liés au caractère mâle des animaux pratiquement jusqu'à leur abattage par le fait que, avant ou pendant la période d'engraissement des animaux, on leur administre une fois un vaccin anti-LHRH conçu pour induire une première réponse immunitaire de faible intensité, sans effet notable, ou même mesurable, sur la sécrétion des stéroïdes gonadiques et que, seulement peu avant l'abattage, on leur administre un vaccin anti-LHRH pour induire une immunoneutralisation anti-LHRH produisant la suppression ou l'abaissement significatif de la sécrétion des stéroïdes.

2. Procédé selon la revendication 1, caractérisé en ce que le vaccin administré en premier lieu et le vaccin administré peu avant l'abattage sont choisis parmi les vaccins en émulsion et les vaccins en adjuvant aqueux.

3. Procédé selon l'une quelconque des revendications 1 et 2, caractérisé en ce que le vaccin anti-LHRH administré en premier lieu est un vaccin en émulsion.

4. Procédé selon l'une quelconque des revendications 1 à 3, caractérisé en ce que, pour le porc, on administre, peur avant l'abattage, le vaccin anti-LHRH avec un adjuvant de type aqueux.

5. Procédé selon la revendication 4, caractérisé en ce que, comme adjuvant de type aqueux, on utilise du gel d'hydroxyde d'aluminium et/ou de la saponine.

6. Procédé selon la revendication 4 ou 5, caractérisé en ce qu'on administre le vaccin en adjuvant aqueux de 15 à 21 jours avant l'abattage.

7. Procédé selon l'une quelconque des revendications 1 à 3, caractérisé en ce que, pour les bovins et les ovins, on administre, avant l'abattage, un vaccin anti-LHRH avec un adjuvant en émulsion.

8. Procédé selon la revendication 7, caractérisé en ce qu'on administre le vaccin en émulsion de un à deux mois avant l'abattage.

9. Procédé selon la revendication 7 ou 8, caractérisé en ce qu'on administre le vaccin en émulsion de quatre semaines à plusieurs mois après l'administration faite en premier lieu.

10. Procédé selon l'une quelconque des revendications 2, 3 et 7 à 9, caractérisé en ce que le vaccin en émulsion est un vaccin en émulsion eau-dans-l'huile.

11. Procédé selon la revendication 10, caractérisé en ce que l'émulsion eau-dans-l'huile est faite d'un mélange d'huiles minérales hautement purifiées et de tensioactifs non ioniques.

12. Procédé selon l'une quelconque des revendications 1 à 11, caractérisé en ce que l'on administre un conjugué immunogène anti-LHRH comprenant :
- la LHRH totale ou modifiée,
- un fragment peptidique de LHRH modifié ou non, ou
- un agoniste de la LHRH, couplé à une protéine porteuse immunogène choisie parmi :
- sérum albumine bovine ou humaine,
- thyroglobuline,
- ovalbumine,
- les anatoxines, notamment l'anatoxine tétanique,
- globulines humaines ou équines.

13. Procédé selon la revendication 12, caractérisé en ce que le conjugué comprend la LHRH couplée à l'alpha-globuline équine, notamment fractions IV-1 et/ou IV-4.

14. Procédé selon la revendication 12, caractérisé en ce que le conjugué comprend la LHRH (3-10) couplée à l'alpha-globuline équine, notamment les fractions IV-1 et/ou IV-4, ou à l'ovalbumine.

15. Procédé selon la revendication 13 ou 14, caractérisé en ce que la LHRH/LHRH (3-10) et la protéine porteuse immunogène sont couplées par un carbodiimide.

16. Procédé selon la revendication 1, caractérisé en ce que, au lieu de vaccin pour immunoneutralisation active, on administre à l'animal, quelques jours avant l'abattage, du sérum ou du plasma hyperimmun anti-LHRH.

17. Procédé selon la revendication 1, caractérisé en ce que, au lieu de vaccin pour immunoneutralisation active, on administre à l'animal, quelques jours avant l'abattage, des anticorps monoclonaux anti-LHRH.

18. Procédé selon la revendication 16 ou 17, caractérisé en ce que l'administration est faite de cinq à quinze jours avant l'abattage, par voie sous-cutanée ou intramusculaire.

19. Procédé selon l'une quelconque des revendications 1 à 16, caractérisé en ce qu'on administre par voie transcutanée, de préférence en plusieurs points, à l'aide d'un appareil d'injection sans aiguille par jet sous pression.

20. Procédé selon l'une quelconque des revendications 1 à 19, caractérisé en ce que le vaccin anti-LHRH administré en premier lieu l'est avant la phase d'engraissement de l'animal.

21. Procédé de préparation d'un ensemble de vaccination anti-LHRH utilisable pour la production de viande ayant des qualités organoleptiques améliorées à partir de porcins, bovins ou ovins mâles non castrés, procédé dans lequel on prépare :
- un vaccin anti-LHRH conçu pour être administré en premier lieu avant ou pendant la phase d'engraissement des animaux et conçu pour induire une première réponse immunitaire de faible intensité, sans effet notable, ou même mesurable, sur la sécrétion des stéroides gonadiques, pour permettre le développement du caractère mâle des animaux, et
- un vaccin anti-LHRH conçu pour être administré après le précédent et seulement peut avant l'abattage, ce vaccin étant conçu pour induire une immunoneutralisation anti-LHRH produisant la suppression ou l'abaissement significatif de la sécrétion des stéroides.

22. Procédé selon la revendication 21, caractérisé en ce que l'on réalise un conjugué entre une protéine porteuse immunogène et un peptide LHRH pour former le principe actif des vaccins.

23. Procédé selon la revendication 22, caractérisé en ce que le peptide LHRH est choisi dans le groupe consistant en :
- LHRH totale ou modifiée,
- fragment peptidique de LHRH, modifié ou non,
- agoniste de LHRH.

24. Procédé selon la revendication 22 ou 23, caractérisé en ce que la protéine porteuse immunogène est choisie dans le groupe consistant en :
- sérum albumine,
- thyroglobuline,
- ovalbumine
- anatoxines,
- anatoxine tétanique,
- globulines équines,
- globulines humaines,
- alpha-globulines

25. Procédé selon l'une des revendications 21 à 24, caractérisé en ce que le vaccin anti-LHRH à administrer en première injection est préparé sous forme d'émulsion.

26. Procédé selon l'une des revendications 21 à 25, caractérisé en ce que le vaccin anti-LHRH à administrer peu avant l'abattage est préparé sous forme d'émulsion.

27. Procédé selon la revendication 25 ou 26, caractérisé en ce que le vaccin sous forme d'émulsion est préparé sous forme d'émulsion eau-dans-l'huile.

28. Procédé selon l'une des revendications 25 à 27, caractérisé en ce que, pour la préparation du vaccin sous forme d'émulsion, on emploie un mélange d'huiles minérales et de tensioactifs non ioniques.

29. Procédé selon l'une des revendications 21 à 25, caractérisé en ce que, pour le vaccin anti-LHRH destiné à être administré peu avant l'abattage, on ajoute un adjuvant de type aqueux.

30. Procédé selon la revendication 29, caractérisé en ce que l'adjuvant de type aqueux est choisi dans le groupe consistant en gel d'hydroxyde d'aluminium, saponine et mélange de ceux-ci.

31. Procédé selon l'une des revendications 21 à 25 et 29, 30, caractérisé en ce que, pour le vaccin anti-LHRH à administrer peu avant l'abattage chez le porc, on ajoute un adjuvant de type aqueux.

32. Procédé selon l'une des revendications 21 à 28, caractérisé en ce que le vaccin anti-LHRH à administrer peu avant l'abattage chez les bovins et les ovins est préparé sous forme d'émulsion.

33. Procédé selon l'une quelconque des revendications 21 à 32, caractérisé en ce que l'on combine dans un emballage unique, un nombre identique de doses de chacun des vaccins.

34. Procédé selon l'une quelconque des revendications 1 à 11, caractérisé en ce que l'on administre un vaccin comprenant un principe actif immunogène comprenant le peptide LHRH.

35. Procédé selon la revendication 21 ou selon l'une quelconque des revendications 25 à 33 ensemble la revendication 21, caractérisé en ce que l'on utilise un principe actif immunogène comprenant le peptide LHRH pour la préparation des vaccins anti-LHRH.

## Claims (Claims for the following Contracting State(s): AT, BE, CH, LI, DE, DK, FR, GB, IT, LU, NL, PT)

1. Process for producing pork, beef or sheep meat having improved organoleptic qualities, particularly improved smell, juiciness and tenderness, wherein uncastrated male domestic animals are fattened and the animals are given active immunoneutralisation using an anti-LHRH vaccine, characterised in that the advantages connected with the male nature of the animals are maintained virtually until they are slaughtered by the fact that, before or during the period of fattening the animals, they are given a single anti-LHRH vaccine designed to induce a first low-intensity immune response, with no appreciable or even measurable effect on the secretion of sex steroids and, just before slaughtering, they are given an anti-LHRH vaccine to induce anti-LHRH immunoneutralisation which leads to suppression or a significant reduction in the secretion of steroids.

2. Process according to claim 1, characterised in that the vaccine administered in the first place and the vaccine administered shortly before slaughtering are selected from emulsified vaccines and vaccines in an aqueous adjuvant.

3. Process according to either of claims 1 and 2, characterised in that the anti-LHRH vaccine administered in the first place is an emulsified vaccine.

4. Process according to any one of claims 1 to 3, characterised in that, for pork, the anti-LHRH vaccine is administered with an aqueous adjuvant, shortly before slaughtering.

5. Process according to claim 4, characterised in that aluminium hydroxide gel and/or saponin is used as the aqueous adjuvant.

6. Process according to claims 4 or 5, characterised in that the vaccine is administered in an aqueous adjuvant 15 to 21 days before slaughtering.

7. Process according to any one of claims 1 to 3, characterised in that, for cattle and sheep, an anti-LHRH vaccine is administered with an emulsified adjuvant before slaughtering.

8. Process according to claim 7, characterised in that the emulsified vaccine is administered one to two months before slaughtering.

9. Process according to claims 7 or 8, characterised in that the emulsified vaccine is administered four weeks to several months after the initial administration.

10. Process according to any one of claims 2, 3 and 7 to 9, characterised in that the emulsified vaccine is a vaccine in a water-in-oil emulsion.

11. Process according to claim 10, characterised in that the water-in-oil emulsion is made from a mixture of highly purified mineral oils and non-ionic surfactants.

12. Process according to any one of claims 1 to 11, characterised in that an anti-LHRH immunogenic conjugate is administered comprising:
- total or modified LHRH,
- a peptide fragment of modified or unmodified LHRH, or
- an LHRH agonist,
- coupled with an immunogenic carrier protein selected from among:
- bovine or human serum albumin,
- thyroglobulin,
- ovalbumin,
- the anatoxins, notably tetanus anatoxin,
- human or equine globulins.

13. Process according to claim 12, characterised in that the conjugate comprises LHRH coupled with equine alpha globulin, especially fractions IV-1 and/or IV-4.

14. Process according to claim 12, characterised in that the conjugate comprises LHRH (3-10) coupled with equine alpha globulin, notably fractions IV-1 and/or IV-4, or with ovalbumin.

15. Process according to claims 13 or 14, characterised in that the LHRH/LHRH (3-10) and the immunogenic carrier protein are coupled by a carbodiimide.

16. Process according to claim 1, characterised in that anti-LHRH serum or hyperimmune plasma is administered to the animal, some days before slaughtering.

17. Process according to claim 1, characterised in that anti-LHRH monoclonal antibodies are administered to the animal several days before slaughtering.

18. Process according to claims 16 or 17, characterised in that the substance is administered five to fifteen days before slaughtering, by subcutaneous or intramuscular route.

19. Process according to any one of claims 1 to 18, characterised in that the substance is administered by transcutaneous route, preferably at several points, using a needleless pressure jet injection apparatus.

20. Process according to any one of claims 1 to 19, characterised in that the anti-LHRH vaccine administered in the first place is given before the stage of fattening the animal.

21. Anti-LHRH vaccination kit which can be used for the production of meat having improved organoleptic qualities from uncastrated male pigs, cattle or sheep, this vaccination kit comprising:
- an anti-LHRH vaccine designed to be given in the first place before or during the stage of fattening the animals and designed to induce a first low intensity immune response with no appreciable or even measurable effect on the secretion of the sex steroids, to allow the male nature of the animals to develop, and
- an anti-LHRH vaccine designed to be administered after the previous one and just a short time before slaughtering, this vaccine being designed to induce anti-LHRH immunoneutralisation causing the suppression or significant lowering of the secretion of steroids.

22. Vaccination kit according to claim 21, characterised in that the vaccines contain an active principle which is a conjugate of an immunogenic carrier protein and an LHRH peptide.

23. Vaccination kit according to claim 22, characterised in that the LHRH peptide is selected from the group consisting of:
- total or modified LHRH,
- peptide fragment of LHRH, which may or may not be modified,
- LHRH agonist.

24. Vaccination kit according to claim 22 or 23, characterised in that the immunogenic carrier protein is selected from the group comprising:
- serum albumin,
- thyroglobulin,
- ovalbumin,
- anatoxins,
- tetanus anatoxin,
- equine globulins,
- human globulins,
- alpha globulins.

25. Vaccination kit according to one of claims 21 to 24, characterised in that the anti-LHRH vaccine to be administered by the first injection is a vaccine in the form of an emulsion.

26. Vaccination kit according to one of claims 21 to 25, characterised in that the anti-LHRH vaccine to be administered shortly before slaughtering is a vaccine in the form of an emulsion.

27. Vaccination kit according to claim 25 or 26, characterised in that the vaccine in the form of an emulsion is a vaccine in the form of a water-in-oil emulsion.

28. Vaccination kit according to one of claims 25 to 27, characterised in that the vaccine in the form of an emulsion is made up of a mixture of mineral oils and non-ionic surfactants.

29. Vaccination kit according to one of claims 21 to 25, characterised in that the anti-LHRH vaccine intended to be administered shortly before slaughtering is a vaccine with an aqueous-type adjuvant.

30. Vaccination kit according to claim 29, characterised in that the aqueous-type adjuvant is selected from the group consisting of aluminium hydroxide gel, saponin and mixtures thereof.

31. Vaccination kit according to one of claims 21 to 25 and 29, 30, intended for pigs, characterised in that the anti-LHRH vaccine to be administered shortly before slaughtering is a vaccine with an aqueous-type adjuvant.

32. Vaccination kit according to one of claims 21 to 28 for use in cattle and sheep, characterised in that the anti-LHRH vaccine to be administered shortly before slaughtering is a vaccine in the form of an emulsion.

33. Vaccination kit according to any one of claims 21 to 32, characterised in that the vaccination kit consists of an identical number of doses of each of the vaccines in a single package.

34. Process according to any one of claims 1 to 11, characterised in that a vaccine is administered containing an immunogenic active principle which comprises the LHRH peptide.

35. Vaccination kit according to claim 21 or any one of claims 25 to 33 together with claim 21, characterised in that the anti-LHRH vaccines comprise an immunogenic active principle containing the peptide LHRH.

## Claims (Claims for the following Contracting State(s): ES, GR)

1. Process for producing pork, beef or sheep meat having improved organoleptic qualities, particularly improved smell, juiciness and tenderness, wherein uncastrated male domestic animals are fattened and the animals are given active immunoneutralisation using an anti-LHRH vaccine, characterised in that the advantages connected with the male nature of the animals are maintained virtually until they are slaughtered by the fact that, before or during the period of fattening the animals, they are given a single anti-LHRH vaccine designed to induce a first low-intensity immune response, with no appreciable or even measurable effect on the secretion of sex steroids and, just before slaughtering, they are given an anti-LHRH vaccine to induce anti-LHRH immunoneutralisation which leads to suppression or a significant reduction in the secretion of steroids.

2. Process according to claim 1, characterised in that the vaccine administered in the first place and the vaccine administered shortly before slaughtering are selected from emulsified vaccines and vaccines in an aqueous adjuvant.

3. Process according to either of claims 1 and 2, characterised in that the anti-LHRH vaccine administered in the first place is an emulsified vaccine.

4. Process according to any one of claims 1 to 3, characterised in that, for pork, the anti-LHRH vaccine is administered with an aqueous adjuvant, shortly before slaughtering.

5. Process according to claim 4, characterised in that aluminium hydroxide gel and/or saponin is used as the aqueous adjuvant.

6. Process according to claims 4 or 5, characterised in that the vaccine is administered in an aqueous adjuvant 15 to 21 days before slaughtering.

7. Process according to any one of claims 1 to 3, characterised in that, for cattle and sheep, an anti-LHRH vaccine is administered with an emulsified adjuvant before slaughtering.

8. Process according to claim 7, characterised in that the emulsified vaccine is administered one to two months before slaughtering.

9. Process according to claims 7 or 8, characterised in that the emulsified vaccine is administered four weeks to several months after the initial administration.

10. Process according to any one of claims 2, 3 and 7 to 9, characterised in that the emulsified vaccine is a vaccine in a water-in-oil emulsion.

11. Process according to claim 10, characterised in that the water-in-oil emulsion is made from a mixture of highly purified mineral oils and non-ionic surfactants.

12. Process according to any one of claims 1 to 11, characterised in that an anti-LHRH immunogenic conjugate is administered comprising:
- total or modified LHRH,
- a peptide fragment of modified or unmodified LHRH, or
- an LHRH agonist,
- coupled with an immunogenic carrier protein selected from among:
- bovine or human serum albumin,
- thyroglobulin,
- ovalbumin,
- the anatoxins, notably tetanus anatoxin,
- human or equine globulins.

13. Process according to claim 12, characterised in that the conjugate comprises LHRH coupled with equine alpha globulin, especially fractions IV-1 and/or IV-4.

14. Process according to claim 12, characterised in that the conjugate comprises LHRH (3-10) coupled with equine alpha globulin, notably fractions IV-1 and/or IV-4, or with ovalbumin.

15. Process according to claims 13 or 14, characterised in that the LHRH/LHRH (3-10) and the immunogenic carrier protein are coupled by a carbodiimide.

16. Process according to claim 1, characterised in that anti-LHRH serum or hyperimmune plasma is administered to the animal, some days before slaughtering.

17. Process according to claim 1, characterised in that anti-LHRH monoclonal antibodies are administered to the animal several days before slaughtering.

18. Process according to claims 16 or 17, characterised in that the substance is administered five to fifteen days before slaughtering, by subcutaneous or intramuscular route.

19. Process according to any one of claims 1 to 18, characterised in that the substance is administered by transcutaneous route, preferably at several points, using a needleless pressure jet injection apparatus.

20. Process according to any one of claims 1 to 19, characterised in that the anti-LHRH vaccine administered in the first place is given before the stage of fattening the animal.

21. Process for preparing an anti-LHRH vaccination kit which can be used for the production of meat having improved organoleptic qualities from uncastrated male pigs, cattle or sheep, wherein one prepares.
- an anti-LHRH vaccine designed to be given in the first place before or during the stage of fattening the animals and designed to induce a first low intensity immune response with no appreciable or even measurable effect on the secretion of the sex steroids, to allow the male nature of the animals to develop, and
- an anti-LHRH vaccine designed to be administered after the previous one and just a short time before slaughtering, this vaccine being designed to induce anti-LHRH immunoneutralisation causing the suppression or significant lowering of the secretion of steroids.

22. Process according to claim 21, characterised in that a conjugate of an immunogenic carrier protein and an LHRH peptide is prepared to form the active principle of the vaccine.

23. Process according to claim 22, characterised in that the LHRH peptide is selected from the group consisting of:
- total or modified LHRH,
- peptide fragment of LHRH, which may or may not be modified,
- LHRH agonist.

24. Process according to claim 22 or 23, characterised in that the immunogenic carrier protein is selected from the group comprising:
- serum albumin,
- thyroglobulin,
- ovalbumin,
- anatoxins,
- tetanus anatoxin,
- equine globulins,
- human globulins,
- alpha globulins.

25. Process according to one of claims 21 to 24, characterised in that the anti-LHRH vaccine to be administered by the first injection is prepared in the form of an emulsion.

26. Process according to one of claims 21 to 25, characterised in that the anti-LHRH vaccine to be administered shortly before slaughtering is prepared in the form of an emulsion.

27. Process according to claim 25 or 26, characterised in that the vaccine in the form of an emulsion is prepared in the form of a water-in-oil emulsion.

28. Process according to one of claims 25 to 27, characterised in that in order to prepare the vaccine in the form of an emulsion, a mixture of mineral oils and non-ionic surfactants is used.

29. Process according to one of claims 21 to 25, characterised in that an aqueous-type adjuvant is added for the anti-LHRH vaccine intended to be administered shortly before slaughtering.

30. Process according to claim 29, characterised in that the aqueous-type adjuvant is selected from the group consisting of aluminium hydroxide gel, saponin and mixtures thereof.

31. Process according to one of claims 21 to 25 and 29, 30, intended for pigs, characterised in that an aqueous-type adjuvant is added for the anti-LHRH vaccine to be administered shortly before slaughtering.

32. Process according to one of claims 21 to 28 for use in cattle and sheep, characterised in that the anti-LHRH vaccine to be administered shortly before slaughtering is prepared in the form of an emulsion.

33. Process according to any one of claims 21 to 32, characterised in that an identical number of doses of each of the vaccines is combined in a single package.

34. Process according to any one of claims 1 to 11, characterised in that a vaccine is administered containing an immunogenic active principle which comprises the LHRH peptide.

35. Process according to claim 21 or any one of claims 25 to 33 together with claim 21, characterised in that for the preparation of the anti-LHRH vaccines an immunogenic active principle containing the peptide LHRH is used.

## Patentansprüche (Patentansprüche für folgende(n) Vertragsstaat(en): AT, BE, CH, LI, DE, DK, FR, GB, IT, LU, NL, PT)

1. Verfahren zur Herstellung von Schweine-, Rind-, Schaffleisch mit verbesserten organoleptischen Eigenschaften, insbesondere Geruch, Geschmack und Zartheit, wobei männliche nicht-kastrierte Haustiere gemästet werden, eine aktive Immunoneutralisierung mit einem anti-LHRH-Impfstoff bei den Tieren vorgenommen wird, dadurch gekennzeichnet, dass man die mit dem männlichen Charakter der Tiere einhergehenden Vorteile praktisch bis zu ihrer Schlachtung dadurch erhält, dass ihnen vor oder während des Mästzeitraums der Tiere einmal ein anti-LHRH-Impfstoff verabreicht wird, der so konzipiert ist, dass er eine erste Immunantwort mit schwacher Intensität, ohne merkliche oder messbare Wirkung auf die Sekretion von Gonadensteroiden hervorruft, und dass man ihnen nur kurz vor der Schlachtung einen anti-LHRH-Impfstoff verabreicht, um eine anti-LHRH-Immunoneutralisation zu induzieren, welche die Unterdrückung oder signifikante Senkung der Sekretion von Steroiden hervorruft.

2. Verfahren nach Anspruch 1, dadurch gekennzeichnet, dass der zuerst verabreichte Impfstoff und der kurz vor der Schlachtung verabreichte Impfstoff ausgewählt sind aus den Emulsionsimpfstoffen und den Impfstoffen mit wässrigem Adjuvans.

3. Verfahren nach einem der Ansprüche 1 und 2, dadurch gekennzeichnet, dass der zuerst verabreichte anti-LHRH-Impfstoff ein Emulsionsimpfstoff ist.

4. Verfahren nach einem der Ansprüche 1 bis 3, dadurch gekennzeichnet, dass man beim Schwein kurz vor der Schlachtung den anti-LHRH-Impfstoff mit wässrigem Adjuvans verabreicht.

5. Verfahren nach Anspruch 4, dadurch gekennzeichnet, dass als wässriges Adjuvans Aluminiumhydroxidgel und/oder Saponin verwendet wird.

6. Verfahren nach Anspruch 4 oder 5, dadurch gekennzeichnet, dass der Impfstoff mit wässrigem Adjuvans 15 bis 21 Tage vor der Schlachtung verabreicht wird.

7. Verfahren nach einem der Ansprüche 1 bis 3, dadurch gekennzeichnet, dass für Rinder und Schafe vor der Schlachtung ein anti-LHRH-Impfstoff mit einem Emulsionsadjuvans verabreicht wird.

8. Verfahren nach Anspruch 7, dadurch gekennzeichnet, dass der Emulsionsimpfstoff ein bis zwei Monate vor der Schlachtung verabreicht wird.

9. Verfahren nach Anspruch 7 oder 6, dadurch gekennzeichnet, dass der Emulsionsimpfstoff vier Wochen bis mehrere Monate nach der zuerst durchgeführten Verabreichung verabreicht wird.

10. Verfahren nach einem der Ansprüche 2, 3 und 7 bis 9, dadurch gekennzeichnet, dass der Emulsionsimpfstoff ein Wasser-in-Öl-Emulsionsimpfstoff ist.

11. Verfahren nach Anspruch 10, dadurch gekennzeichnet, dass die Wasser-in-Öl-Emulsion aus einem Gemisch hochgereinigter Mineralöle und nichtionischer Tenside hergestellt wird.

12. Verfahren nach einem der Ansprüche 1 bis 11, dadurch gekennzeichnet, dass ein anti-LHRH-Immunogen-Konjugat verabreicht wird, umfassend:
- das gesamte oder modifizierte LHRH,
- ein modifiziertes oder unmodifiziertes LHRH-Peptidfragment oder
- einen LHRH-Agonisten,
gekoppelt an ein immunogenes Trägerprotein, ausgewählt aus:
- Rinder- oder Menschen-Serumalbumin,
- Thyreoglobulin,
- Ovalbumin,
- den Anatoxinen, insbesondere dem Tetanus-Anatoxin,
- menschlichen oder Pferde-Globulinen.

13. Verfahren nach Anspruch 12, dadurch gekennzeichnet, dass das Konjugat LHRH, gekoppelt an Pferde-Alphaglobulin, insbesondere die Fraktionen IV-1 und/oder IV-4, umfasst.

14. Verfahren nach Anspruch 12, dadurch gekennzeichnet, dass das Konjugat LHRH (3-10), gekoppelt an Pferde-Alphaglobulin, insbesondere die Fraktionen IV-1 und/oder IV-4, oder an Ovalbumin, umfasst.

15. Verfahren nach Anspruch 13 oder 14, dadurch gekennzeichnet, dass LHRH/LHRH (3-10) und das immunogene Trägerprotein durch ein Carbodiimid gekuppelt sind.

16. Verfahren nach Anspruch 1, dadurch gekennzeichnet, dass man dem Tier mehrere Tage vor der Schlachtung hyperimmunes anti-LHRH-Serum oder -Plasma verabreicht.

17. Verfahren nach Anspruch 1, dadurch gekennzeichnet, dass man dem Tier mehrere Tage vor der Schlachtung monoklonale anti-LHRH-Antikörper verabreicht.

18. Verfahren nach Anspruch 16 oder 17, dadurch gekennzeichnet, dass die Verabreichung fünf bis fünfzehn Tage vor der Schlachtung auf subkutanem oder intramuskulärem Weg erfolgt.

19. Verfahren nach einem der Ansprüche 1 bis 18, dadurch gekennzeichnet, dass man die Verabreichung auf transkutanem Weg, vorzugsweise an mehreren Stellen, mittels einer Injektionsvorrichtung ohne Nadel durch Druckstrahl durchführt.

20. Verfahren nach einem der Ansprüche 1 bis 19, dadurch gekennzeichnet, dass der zuerst verabreichte anti-LHRH-Impfstoff vor der Mästperiode des Tiers verabreicht wird.

21. Kit zur anti-LHRH-Impfung, das sich zur Herstellung von Fleisch mit verbesserten organoleptischen Eigenschaften ausgehend von männlichen, nicht-kastrierten Schweinen, Rindern oder Schafen eignet, wobei das Kit umfasst:
- einen anti-LHRH-Impfstoff zur ersten Verabreichung vor oder während der Mästphase der Tiere, der so konzipiert ist, dass er eine erste Immunantwort mit schwacher Intensität, ohne merkliche oder messbare Wirkung auf die Sekretion von Gonadensteroiden hervorruft, so dass die Entwicklung des männlichen Charakters der Tiere ermöglicht wird, und
- einen anti-LHRH-Impfstoff zur Verabreichung nach dem ersten Impfstoff und nur kurz vor der Schlachtung, wobei dieser Impfstoff so konzipiert ist, dass er eine anti-LHRH-Immunoneutralisation induziert, welche die Unterdrückung oder signifikante Senkung der Sekretion von Steroiden hervorruft.

22. Kit zur Impfung nach Anspruch 21, dadurch gekennzeichnet, dass die Impfstoffe einen Wirkstoff umfassen, der ein Konjugat zwischen einem immunogenen Trägerprotein und einem LHRH-Peptid ist.

23. Kit zur Impfung nach Anspruch 22, dadurch gekennzeichnet, dass das LHRH-Peptid ausgewählt ist aus der Gruppe, bestehend aus:
- gesamtem oder modifiziertem LHRH,
- einem modifizierten oder unmodifizierten LHRH-Peptidfragment,
- einem LHRH-Agonisten.

24. Kit zur Impfung nach Anspruch 22 oder 23, dadurch gekennzeichnet, dass das immunogene Trägerprotein ausgewählt ist aus der Gruppe, bestehend aus:
- Serumalbumin,
- Thyreoglobulin,
- Ovalbumin,
- Anatoxinen,
- Tetanus-Anatoxin,
- Pferde-Globulinen,
- menschlichen Globulinen,
- Alpha-Globulinen.

25. Kit zur Impfung nach einem der Ansprüche 21 bis 24, dadurch gekennzeichnet, dass der bei der ersten Injektion zu injizierende anti-LHRH-Impfstoff ein Impfstoff in Emulsionsform ist.

26. Kit zur Impfung nach einem der Ansprüche 21 bis 25, dadurch gekennzeichnet, dass der kurz vor der Schlachtung zu verabreichende anti-LHRH-Impfstoff ein Impfstoff in Emulsionsform ist.

27. Kit zur Impfung nach Anspruch 25 oder 26, dadurch gekennzeichnet, dass der Impfstoff in Emulsionsform ein Impfstoff in Form einer Wasser-in-Öl-Emulsion ist.

28. Kit zur Impfung nach einem der Ansprüche 25 bis 27, dadurch gekennzeichnet, dass der Impfstoff in Emulsionsform aus einem Gemisch von Mineralölen und nichtionischen Tensiden hergestellt ist.

29. Kit zur Impfung nach einem der Ansprüche 21 bis 25, dadurch gekennzeichnet, dass der für die Verabreichung kurz vor der Schlachtung bestimmte anti-LHRH-Impfstoff ein Impfstoff mit einem wässrigen Adjuvans ist.

30. Kit zur Impfung nach Anspruch 29, dadurch gekennzeichnet, dass das wässrige Adjuvans ausgewählt ist aus der Gruppe, bestehend aus Aluminiumhydroxidgel, Saponin und einem Gemisch davon.

31. Kit zur Impfung nach einem der Ansprüche 21 bis 25 und 29, 30, das für Schweine bestimmt ist, dadurch gekennzeichnet, dass der kurz vor der Schlachtung zu verabreichende anti-LHRH-Impfstoff ein Impfstoff mit einen wässrigen Adjuvans ist.

32. Kit zur Impfung nach einem der Ansprüche 21 bis 28, das für Rinder und Schafe anwendbar ist, dadurch gekennzeichnet, dass der kurz vor der Schlachtung zu verabreichende anti-LHRH-Impfstoff ein Impfstoff in Emulsionsform ist.

33. Kit zur Impfung nach einem der Ansprüche 21 bis 32, dadurch gekennzeichnet, dass das Impf-Kit aus einer identischen Anzahl an Dosierungen von jedem Impfstoff in einer einzelnen Verpackung besteht.

34. Verfahren nach einem der Ansprüche 1 bis 11, dadurch gekennzeichnet, dass ein Impfstoff verabreicht wird, umfassend einen immunogenen Wirkstoff, der das LHRH-Peptid umfasst.

35. Kit zur Impfung nach Anspruch 21 oder nach einem der Ansprüche 25 bis 33 zusammen mit Anspruch 21, dadurch gekennzeichnet, dass die anti-LHRH-Impfstoffe einen immunogenen Wirkstoff umfassen, der das LHRH-Peptid umfasst.

## Patentansprüche (Patentansprüche für folgende(n) Vertragsstaat(en): ES, GR)

1. Verfahren zur Herstellung von Schweine-, Rind-, Schaffleisch mit verbesserten organoleptischen Eigenschaften, insbesondere Geruch, Geschmack und Zartheit, wobei männliche nicht-kastrierte Haustiere gemästet werden, eine aktive Immunoneutralisierung mit einem anti-LHRH-Impfstoff bei den Tieren vorgenommen wird, dadurch gekennzeichnet, dass man die mit dem männlichen Charakter der Tiere einhergehenden Vorteile praktisch bis zu ihrer Schlachtung dadurch erhält, dass ihnen vor oder während des Mästzeitraums der Tiere einmal ein anti-LHRH-Impfstoff verabreicht wird, der so konzipiert ist, dass er eine erste Immunantwort mit schwacher Intensität, ohne merkliche oder messbare Wirkung auf die Sekretion von Gonadensteroiden hervorruft, und dass man ihnen nur kurz vor der Schlachtung einen anti-LHRH-Impfstoff verabreicht, um eine anti-LHRH-Immunoneutralisation zu induzieren, welche die Unterdrückung oder signifikante Senkung der Sekretion von Steroiden hervorruft.

2. Verfahren nach Anspruch 1, dadurch gekennzeichnet, dass der zuerst verabreichte Impfstoff und der kurz vor der Schlachtung verabreichte Impfstoff ausgewählt sind aus den Emulsionsimpfstoffen und den Impfstoffen mit wässrigem Adjuvans.

3. Verfahren nach einem der Ansprüche 1 und 2, dadurch gekennzeichnet, dass der zuerst verabreichte anti-LHRH-Impfstoff ein Emulsionsimpfstoff ist.

4. Verfahren nach einem der Ansprüche 1 bis 3, dadurch gekennzeichnet, dass man beim Schwein kurz vor der Schlachtung den anti-LHRH-Impfstoff mit wässrigem Adjuvans verabreicht.

5. Verfahren nach Anspruch 4, dadurch gekennzeichnet, dass als wässriges Adjuvans Aluminiumhydroxidgel und/oder Saponin verwendet wird.

6. Verfahren nach Anspruch 4 oder 5, dadurch gekennzeichnet, dass der Impfstoff mit wässrigem Adjuvans 15 bis 21 Tage vor der Schlachtung verabreicht wird.

7. Verfahren nach einem der Ansprüche 1 bis 3, dadurch gekennzeichnet, dass für Rinder und Schafe vor der Schlachtung ein anti-LHRH-Impfstoff mit einem Emulsionsadjuvans verabreicht wird.

8. Verfahren nach Anspruch 7, dadurch gekennzeichnet, dass der Emulsionsimpfstoff ein bis zwei Monate vor der Schlachtung verabreicht wird.

9. Verfahren nach Anspruch 7 oder 8, dadurch gekennzeichnet, dass der Emulsionsimpfstoff vier Wochen bis mehrere Monate nach der zuerst durchgeführten Verabreichung verabreicht wird.

10. Verfahren nach einem der Ansprüche 2, 3 und 7 bis 9, dadurch gekennzeichnet, dass der Emulsionsimpfstoff ein Wasser-in-Öl-Emulsionsimpfstoff ist.

11. Verfahren nach Anspruch 10, dadurch gekennzeichnet, dass die Wasser-in-Öl-Emulsion aus einem Gemisch hochgereinigter Mineralöle und nichtionischer Tenside hergestellt wird.

12. Verfahren nach einem der Ansprüche 1 bis 11, dadurch gekennzeichnet, dass ein anti-LHRH-Immunogen-Konjugat verabreicht wird, umfassend:
- das gesamte oder modifizierte LHRH,
- ein modifiziertes oder unmodifiziertes LHRH-Peptidfragment oder
- einen LHRH-Agonisten,
gekoppelt an ein immunogenes Trägerprotein, ausgewählt aus:
- Rinder- oder Menschen-Serumalbumin,
- Thyreoglobulin,
- Ovalbumin,
- den Anatoxinen, insbesondere dem Tetanus-Anatoxin,
- menschlichen oder Pferde-Globulinen.

13. Verfahren nach Anspruch 12, dadurch gekennzeichnet, dass das Konjugat LHRH, gekoppelt an Pferde-Alphaglobulin, insbesondere die Fraktionen IV-1 und/oder IV-4, umfasst.

14. Verfahren nach Anspruch 12, dadurch gekennzeichnet, dass das Konjugat LHRH (3-10), gekoppelt an Pferde-Alphaglobulin, insbesondere die Fraktionen IV-1 und/oder IV-4, oder an Ovalbumin, umfasst.

15. Verfahren nach Anspruch 13 oder 14, dadurch gekennzeichnet, dass LHRH/LHRH (3-10) und das immunogene Trägerprotein durch ein Carbodiimid gekuppelt sind.

16. Verfahren nach Anspruch 1, dadurch gekennzeichnet, dass man dem Tier mehrere Tage vor der Schlachtung hyperimmunes anti-LHRH-Serum oder -Plasma verabreicht.

17. Verfahren nach Anspruch 1, dadurch gekennzeichnet, dass man dem Tier mehrere Tage vor der Schlachtung monoklonale anti-LHRH-Antikörper verabreicht.

18. Verfahren nach Anspruch 16 oder 17, dadurch gekennzeichnet, dass die Verabreichung fünf bis fünfzehn Tage vor der Schlachtung auf subkutanem oder intramuskulärem Weg erfolgt.

19. Verfahren nach einem der Ansprüche 1 bis 18, dadurch gekennzeichnet, dass man die Verabreichung auf transkutanem Weg, vorzugsweise an mehreren Stellen, mittels einer Injektionsvorrichtung ohne Nadel durch Druckstrahl durchführt.

20. Verfahren nach einem der Ansprüche 1 bis 19, dadurch gekennzeichnet, dass der zuerst verabreichte anti-LHRH-Impfstoff vor der Mästperiode des Tiers verabreicht wird.

21. Verfahren zur Herstellung eines Kits zur anti-LHRH-Impfung, das sich zur Herstellung von Fleisch mit verbesserten organoleptischen Eigenschaften ausgehend von männlichen, nicht-kastrierten Schweinen, Rindern oder Schafen eignet, wobei man :
- einen anti-LHRH-Impfstoff zur ersten Verabreichung vor oder während der Mästphase der Tiere, der so konzipiert ist, dass er eine erste Immunantwort mit schwacher Intensität, ohne merkliche oder messbare Wirkung auf die Sekretion von Gonadensteroiden hervorruft, so dass die Entwicklung des männlichen Charakters der Tiere ermöglicht wird, und
- einen anti-LHRH-Impfstoff zur Verabreichung nach dem ersten Impfstoff und nur kurz vor der Schlachtung, wobei dieser Impfstoff so konzipiert ist, dass er eine anti-LHRH-Immunoneutralisation induziert, welche die Unterdrückung oder signifikante Senkung der Sekretion von Steroiden hervorruft, herstellt.

22. Verfahren nach Anspruch 21, dadurch gekennzeichnet, dass der Wirkstoff für die Impfstoffe aus einem Konjugat zwischen einen immunogenen Trägerprotein und einem LHRH-Peptid hergestellt wird.

23. Verfahren nach Anspruch 22, dadurch gekennzeichnet, dass das LHRH-Peptid ausgewählt ist aus der Gruppe, bestehend aus: - gesamtem oder modifiziertem LHRH, - einem modifizierten oder unmodifizierten LHRH-Peptidfragment, - einem LHRH-Agonisten.

24. Verfahren nach Anspruch 22 oder 23, dadurch gekennzeichnet, dass das immunogene Trägerprotein ausgewählt ist aus der Gruppe, bestehend aus:
- Serumalbumin,
- Thyreoglobulin,
- Ovalbumin,
- Anatoxinen,
- Tetanus-Anatoxin,
- Pferde-Globulinen,
- menschlichen Globulinen,
- Alpha-Globulinen.

25. Verfahren nach einem der Ansprüche 21 bis 24, dadurch gekennzeichnet, dass der bei der ersten Injektion zu injizierende anti-LHRH-Impfstoff aus einen Impfstoff in Emulsionsform hergestellt wird.

26. Verfahren nach einen der Ansprüche 21 bis 25, dadurch gekennzeichnet, dass der kurz vor der Schlachtung zu verabreichende anti-LHRH-Impfstoff als Impfstoff in Emulsionsform vorbereitet wird.

27. Verfahren nach Anspruch 25 oder 26, dadurch gekennzeichnet, dass der Impfstoff in Emulsionsform als Impfstoff in Form einer Wasser-in-Öl-Emulsion verwendet wird.

28. Verfahren nach einem der Ansprüche 25 bis 27, dadurch gekennzeichnet, dass der Impfstoff in Emulsionsform aus einem Gemisch von Mineralölen und nichtionischen Tensiden hergestellt wird.

29. Verfahren nach einem der Ansprüche 21 bis 25, dadurch gekennzeichnet, dass dem für die Verabreichung kurz vor der Schlachtung bestimmten anti-LHRH-Impfstoff ein wässriges Adjuvans zugegeben wird.

30. Verfahren nach Anspruch 29, dadurch gekennzeichnet, dass das wässrige Adjuvans ausgewählt ist aus der Gruppe, bestehend aus Aluminiumhydroxidgel, Saponin und einem Gemisch davon.

31. Verfahren nach einem der Ansprüche 21 bis 25 und 29, 30, dadurch gekennzeichnet, dass den für den für Schweine bestimmten kurz vor der Schlachtung zu verabreichendem anti-LHRH-Impfstoff ein wässriges Adjuvans zugegeben wird.

32. Verfahren nach einem der Ansprüche 21 bis 28, dadurch gekennzeichnet, dass der für Rinder und Schafe anwendbare kurz vor der Schlachtung zu verabreichende anti-LHRH-Impfstoff in Emulsions form vorbereitet wird.

33. Verfahren nach einem der Ansprüche 21 bis 32, dadurch gekennzeichnet, dass das man in einer einzelnen Verpackung eine identische Anzahl an Dosierungen von jedem Impfstoff zusammenstellt.

34. Verfahren nach einem der Ansprüche 1 bis 11, dadurch gekennzeichnet, dass ein Impfstoff verabreicht wird, umfassend einen immunogenen Wirkstoff, der das LHRH-Peptid umfasst.

35. Verfahren nach Anspruch 21 oder nach einem der Ansprüche 25 bis 33 zusammen mit Anspruch 21, dadurch gekennzeichnet, zur Herstellung der anti-LHRH-Impfstoffe ein immunogener Wirkstoff verwendet wird, der das LHRH-Peptid umfasst.
